# EUROPEAN PATENT APPLICATION

(11) **EP 4 353 734 A1**
(43) Date of publication of application: **17.04.2024**
(21) Application number: 22807406.8
(22) Date of filing: 06.05.2022
(51) Int. Cl.: C07K 1/16, C07K 16/18, C07K 17/04, C12N 15/13

(54) **PURIFICATION METHOD OF ANTIBODY COMPOSITION**

(30) Priority: 10.05.2021 JP 2021079977
(71) Applicant: CHIOME BIOSCIENCE INC., Shibuya-ku Tokyo 151-0071 (JP)
(72) Inventor: YOSHIMORI Takayuki, Tokyo 151-0071 (JP); IARUSSO Stefan, 13086 Berlin (DE)
(74) Representative: J A Kemp LLP
(86) International application number: PCT/JP2022/019548
(87) International publication number: WO 2022/239704

(57) **Abstract**

The present invention aims to provide a method for removing glycosylation isomers in antibody drugs, etc. The present invention provides, for example, a purification method for an antibody composition, said method comprising: loading the antibody composition on conventional chromatography to allow an antibody having no sugar chains attached to sites other than the Fc region glycosylation consensus region to be adsorbed to a media of the chromatography; and treating the media with an eluent to thereby elute the antibody adsorbed to the media to obtain a purified antibody composition.

## Description

### Technical Field

The present invention relates to a production method for an antibody composition. In more detail, the present invention relates to a purification method for an antibody composition with reduced levels of isomers having sugar chains attached to sites other than the Fc region glycosylation consensus region.

### Background Art

Antibody drugs comprising a monoclonal antibody as an active ingredient are expected as one of the molecular targeted drugs based on the high binding affinity and binding specificity of an antibody molecule for its antigen, and their research and development have progressed. Antibody drugs are indispensable for the treatment of various diseases including cancers and autoimmune diseases, and nearly 100 of products have now been approved and used in the world (Non-patent Document 1, Non-patent Document 2). Expectations remain high for the development of novel antibody drugs satisfying unmet medical needs, and it is further expected that many new antibody drugs will be investigated and developed.

An antibody (IgG) has an N-linked sugar chain at the Asn residue at position 297 (Asn297) in a glycosylation consensus region (Asn297-X-Ser/Thr, wherein X is an amino acid other than Pro) present in the heavy chain Fc region. Such a sugar chain present in this glycosylation consensus region is known to contribute to properties as an antibody molecule, such as biological activity, pharmacokinetics in blood, safety and so on (Non-patent Document 3, Non-patent Document 4). For example, it has been known that antibody-dependent cellular cytotoxicity (ADCC) activity is enhanced upon removal of the Fuc residue (core fucose) attached to the N-acetylglucosamine (GlcNAc) residue at the reducing end of the N-linked sugar chain at Asn297. Likewise, it has been found that a greater number of galactose (Gal) residues in the non-reducing end portion of a sugar chain linked to the Fc region are more likely to enhance binding to the first complement component (C1q), which in turn enhances complement-dependent cytotoxicity (CDC) activity (Non-patent Document 5).

On the other hand, there are also well-known cases of antibodies (glycosylation isomers) having sugar chains linked to sites other than the glycosylation consensus region in the antibody Fc region. For example, the antineoplastic agent Cetuximab produced in SP2/0 cells was confirmed to have an N-linked sugar chain linked to its Fab region (Non-patent Document 3). In addition to this, various reports have been made on glycosylation isomers (Non-patent Document 6, Non-patent Document 7, Non-patent Document 8).

Glycosylation isomers are known to have the potential to affect various antibody properties such as biological activity (Non-patent Document 10, Non-patent Document 11), immunogenicity (Non-patent Document 8, Non-patent Document 10), blood half-life (Non-patent Document 9), etc. In particular, a sugar chain linked near an Fab region or a CDR region will raise a concern for the risk of reduced biological activity. Likewise, glycosylation isomers, i.e., antibodies having sugar chains linked to sites other than the glycosylation consensus region have a concern for safety such as immunogenicity, etc.

When biological activity is greatly reduced and/or immunogenicity is greatly increased upon attachment of sugar chains to sites other than the glycosylation consensus region, there will arise a negative effect undesirable for ingredients of antibody drugs, so that these glycosylation isomers are regarded as product-related impurities. Such product-related impurities are not desired to be contained as ingredients of drugs, and are desired to be removed as much as possible, also from a regulatory perspective (Non-patent Document 12).

When an antibody comprising a consensus sequence which may have a sugar chain attached to a site other than the glycosylation consensus region in the antibody Fc region is found as a candidate molecule for the development of drugs, it is usual to replace the consensus sequence with another amino acid sequence to ensure that no sugar chains is linked to this site. In this case, binding activity and other properties may be reduced when compared to the performance of the original antibody, and there is a possibility that an antibody having the intended therapeutic effect cannot be obtained.

Glycosylation isomers having sugar chains linked to antibody regions (e.g., Fab or CDR) other than the glycosylation consensus region can be evaluated and distinguished by being separated at the level of small-scale and high-performance analytical methods. There are known cases where peptide fragments are evaluated by high-performance liquid chromatography in combination with mass spectrometry, and where sugar chains cleaved from antibodies are evaluated directly (Non-patent Document 6, Non-patent Document 9). Moreover, another case is known where antibodies having sugar chains linked to their Fab region were analyzed by hydrophobic interaction chromatography (HIC) (Non-patent Document 13). However, these cases were all intended for analytical purpose and designed to use a very small amount of antibody and an analytical method with very high separation performance, and the composition thus separated cannot be used as an antibody drug. Moreover, a review of HIC analysis (Non-patent Document 14) discloses the usefulness of separation performance for many antibodies, but it does not suggest at all that glycosylation isomers can be separated by HIC-based purification techniques.

As described above, no method has been known for separating and removing such glycosylation isomers to prepare an antibody composition free from or with sufficiently reduced levels of isomers having sugar chains attached to sites other than the glycosylation consensus region. Namely, in conventional techniques, glycosylation isomers can merely be evaluated for their glycosylation status at the analytical level, and there has been no method for separating and removing glycosylation isomers of an antibody to produce an antibody composition in an amount sufficient to be provided as a drug. In addition, such an antibody composition has not been known.

In view of the foregoing, in most cases where sugar chains are attached to sites other than the glycosylation consensus region present in the antibody Fc region and such glycosylation isomers are contained, these ingredients are contained as part of antibody drugs without being separated. Cetuximab mentioned above has also been developed as having the composition of a mixture having sugar chains attached to both the glycosylation consensus region in the Fc portion and a non-consensus region within Fab (Non-patent Document 3). If glycosylation isomers have no biological activity, it is not desirable because product-related impurities will be contained as ingredients of drugs.

It should be noted that there are reports on the separation of glycosylation isomers by affinity chromatography with lectin specifically binding to a certain sugar chain (Non-patent Document 15, Non-patent Document 16). This technique is designed to use Concanavalin A as a ligand and has also been found to have high separation properties. However, this technique is affinity-based separation and is not suitable for the production of drugs because of using natural substances. Thus, there is no case where this technique was applied to the production of antibody drugs.

As to techniques known to achieve the separation of sugar chain components in non-antibody proteins, there is a case where genetically recombinant antithrombins were separated by chromatography depending on differences in the number of sugar chains (Patent Document 1). In addition, genetically recombinant erythropoietins and derivatives thereof are separated by chromatography depending on differences in the number of sialic acids added (Patent Document 2). Likewise, there is a case where ovalbumin and transferrin were separated by using isoelectric focusing chromatography (Patent Document 3). However, these techniques cannot be applied directly to antibodies, and there is absolutely no report on a production method designed to separate and remove glycosylation isomers in an antibody composition.

As to techniques other than those mentioned above, it is known that sugar chains are removed by enzymatic treatment (Non-patent Document 17). However, in terms of safety, problems may arise from the separation and removal of enzymes and their origin, etc. Thus, this technique is difficult to use as a production technique for drugs. In the enzymatic removal of antibody sugar chains, it is also impossible to distinguish between sugar chains in glycosylation consensus and non-consensus regions.

The anti-hDLK-1 antibody shown in Patent Document 4 is an antibody whose antitumor activity is expected to be promising.

### Prior Art Documents

### Patent Documents

Patent Document 1: WO2008/120801
Patent Document 2: JP 2001-064300 A
Patent Document 3: WO2005/100379
Patent Document 4: WO2014/054820

### Non-patent Documents

Non-patent Document 1: YAKUGAKU ZASSHI 137(7), 815-816 (2017)
Non-patent Document 2: Bull. Natl. Inst. Health Sci., 132, 36-46 (2014)
Non-patent Document 3: CHROMATOGRAPHY, 34(2), 83-88 (2013)
Non-patent Document 4: Protein Cell, 9, 63-73 (2018)
Non-patent Document 5: Front. Immunol., 8 (646), 1-8 (2017)
Non-patent Document 6: J. Biol. Chem., 284 (47), 32493-32506 (2009)
Non-patent Document 7: Embo J., 10, 2717-2723 (1991)
Non-patent Document 8: J. Immunol., 196, 1435-1441 (2016)
Non-patent Document 9: Anal. Biochem., 349, 197-207 (2006)
Non-patent Document 10: Nature Review Immunology, published online, 04 Feb, (2019)
Non-patent Document 11: Biochem. J., 338 (2), 529-538 (1999)
Non-patent Document 12: ICH Q6 B Guideline
Non-patent Document 13: mAbs, 6 (4), 852-858 (2014)
Non-patent Document 14: J. Pharm. Biomed. Anal., 130, 3-18 (2016)
Non-patent Document 15: J. Biol. Chem., 285 (21), 16012-22 (2010)
Non-patent Document 16: Oncotarget, 7 (21), 31166-76 (2016)
Non-patent Document 17: J. Immunol. Methods, 467, 58-62 (2019)

### Summary of the Invention

### Problem to be Solved by the Invention

Sugar chains linked to sites other than the glycosylation consensus region in an antibody are also undesirable in terms of biological activity and safety, and there has been a demand for the development of techniques by which glycosylation isomers contained in antibody drugs can be removed to sufficient levels and a uniform purified antibody composition can be prepared in a simple manner. Accordingly, the present invention aims to provide a method for removing glycosylation isomers in antibody drugs.

In another aspect, the present invention aims to obtain a more effective and safer purified composition of anti-hDLK-1 antibody.

### Means to Solve the Problem

As shown by the above conventional techniques, there is a report showing that glycosylation isomers were separated by precise and high-resolution chromatography for analysis and characterization purposes, but there has been no report on production techniques or preparation techniques by which glycosylation isomers in antibody drugs can be separated and removed by chromatography. As a result of extensive and intensive efforts made to solve the problems stated above, the inventors of the present invention have surprisingly found that a purified antibody composition with reduced levels of glycosylation isomers can be prepared by using a conventional hydrophobic interaction chromatography media, particularly by optimizing the conditions used for adsorption and separation of glycosylation isomers and a desired product.

Moreover, the inventors of the present invention have made an effort to separate glycosylation isomers and purify non-glycosylation isomers for the anti-hDLK-1 antibody shown in Patent Document 4 by using the newly found method for reducing glycosylation isomers. Glycosylation isomers having sugar chains near CDRs in an antibody may affect the binding activity of the antibody, but the degree of reduction in the activity is also related to the size and positions of sugar chains linked; and hence there is also a possibility that these glycosylation isomers will not affect the activity. Surprisingly, in the anti-hDLK-1 antibody shown in Patent Document 4, the inventors of the present invention have found that glycosylation isomers completely lose activity and therefore become "impurities" in drugs. As a result, the inventors of the present invention have succeeded in identifying glycosylation isomers as new impurities in a crude anti-hDLK-1 antibody product, and have enabled the removal of these glycosylation isomers to thereby achieve the provision of a more effective and safer purified composition of anti-hDLK-1 antibody.

Namely, the present invention relates to (1) to (27) shown below.
(1) A purification method for an anti-hDLK-1 antibody composition, whose heavy chain has an amino acid sequence selected from SEQ ID NOs: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, said method comprising:
   loading a crude antibody product on conventional chromatography to allow an antibody having no sugar chains attached to sites other than the Fc region glycosylation consensus region to be adsorbed to the abovementioned chromatography media; and
   treating the media with an eluent to thereby elute the antibody adsorbed to the media to obtain a purified antibody composition,
   wherein the content of glycosylation isomers having sugar chains attached to sites other than the Fc region glycosylation consensus region in the resulting purified antibody composition is reduced when compared to the crude antibody product.
(2) The purification method according to (1) above, wherein the media of the conventional chromatography is a hydrophobic interaction chromatography media or a mixed-mode chromatography media.
(3) The purification method according to (1) or (2) above, wherein the media of the conventional chromatography has an average particle size of 15 µm or more.
(4) The purification method according to (1) or (2) above, wherein the media of the conventional chromatography has an average particle size of 20 to 100 µm.
(5) The purification method according to any one of (1) to (4) above, wherein the media of the conventional chromatography has a benzyl group or a butyl group.
(6) The purification method according to any one of (1) to (5) above, wherein the protein load per unit volume of the media of the conventional chromatography is 20 mg/mL or more.
(7) The purification method according to any one of (1) to (6) above, wherein the media of the conventional chromatography is a hydrophobic interaction chromatography media, and wherein said method comprises, after loading the crude antibody product on the media, washing the media with a wash buffer before elution.
(8) The purification method according to (7) above, characterized in that the salt concentration of the wash buffer is 10 mM or more higher than the salt concentration of the eluent.
(9) The purification method according to (7) or (8) above, characterized in that the salt concentration of the wash buffer is 0.5 M or more.
(10) The purification method according to (7) or (8) above, characterized in that the salt concentration of the wash buffer is 1.0 M or more.
(11) The purification method according to any one of (7) to (10) above, wherein the pH of the wash buffer is 0.2 Units or more lower than the pH of the eluent and is within the range of pH 4 to 8.
(12) The purification method according to any one of (7) to (11) above, wherein the washing is accomplished by passing two or more column volumes of the wash buffer.
(13) The purification method according to any one of (7) to (12) above, characterized in that the washing and elution are accomplished by using a mobile phase whose pH or/and salt concentration change in a stepwise or linear fashion.
(14) The purification method according to any one of (7) to (13) above, wherein the eluent has a salt concentration of 0.5 M or less or contains no salt and has a pH of 5 to 7.
(15) The purification method according to any one of (1) to (14) above, wherein said method gives a yield of 20% or more.
(16) The purification method according to any one of (1) to (15) above, wherein the ratio of glycosylation isomers relative to the total antibody in the purified antibody composition is 5% or less.
(17) The purification method according to (16) above, wherein the ratio of glycosylation isomers relative to the total antibody in the crude antibody product is higher than 5%.
(18) The purification method for an anti-hDLK-1 antibody composition according to (1) above, said method comprising:
   loading the crude antibody product on conventional chromatography with a media having a benzyl group or a butyl group and having a particle size of 20 to 100 µm;
   allowing the antibody having no sugar chains attached to sites other than the Fc region glycosylation consensus region to be adsorbed tothe media;
   washing the media one or more times with a wash buffer of pH 4 to 6 to remove glycosylation isomers; and
   eluting the antibody adsorbed to the media with an eluent of pH 5 to 7 having a salt concentration of 0.5 M or less or containing no salt to obtain a purified antibody composition,
   wherein the ratio of glycosylation isomers relative to the total antibody in the purified antibody composition is reduced when compared to the crude antibody product.
(19) The purification method for an antibody composition according to (1) above, said method comprising:
   loading the crude antibody product adjusted to a salt concentration of 0.5 M or more on conventional chromatography with a media having a benzyl group or a butyl group and having a particle size of 20 to 100 µm to remove glycosylation isomers into a flow-through fraction;
   washing the media with a wash buffer; and
   eluting the antibody adsorbed to the media with an eluent of pH 5 to 7 having a salt concentration of 0.5 M or less or containing no salt to obtain a purified antibody composition,
   wherein the ratio of glycosylation isomers relative to the total antibody in the purified antibody composition is reduced when compared to the crude antibody product.
(20) The purification method for an antibody composition according to (1) above, said method comprising:
   loading the crude antibody product adjusted to pH 4 to 6 on conventional chromatography with a media having a benzyl group or a butyl group and having a particle size of 20 to 100 µm to remove glycosylation isomers into a flow-through fraction;
   washing the media with a wash buffer; and
   eluting the antibody adsorbed to the media with an eluent of pH 5 to 7 having a salt concentration of 0.5 M or less or containing no salt to obtain a purified antibody composition,
   wherein the ratio of glycosylation isomers relative to the total antibody in the purified antibody composition is reduced when compared to the crude antibody product.
(21) The method according to any one of (1) to (20) above, wherein the protein load per unit volume of the media of the conventional chromatography is 20 g/L or more, and the washing is accomplished by passing five or more column volumes of the wash buffer.
(22) A production method for an antibody composition, which comprises the purification method according to any one of (1) to (21) above, wherein the ratio of an antibody having no sugar chains attached to sites other than the Fc region glycosylation consensus region relative to the total antibody is 95% or more.
(23) A production method for a purified anti-hDLK-1 antibody composition, whose heavy chain has an amino acid sequence selected from SEQ ID Nos: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, said method comprising:
   loading a crude antibody product on conventional chromatography with a media having a benzyl group or a butyl group and having a particle size of 20 to 100 µm;
   allowing an antibody having no sugar chains attached to sites other than the Fc region glycosylation consensus region to be adsorbed to the media;
   washing the media one or more times with a wash buffer containing 0.5 M or more salt to remove glycosylation isomers; and
   eluting the antibody adsorbed to the media with an eluent of pH 5 to 7 having a salt concentration of 0.5 M or less or containing no salt to obtain a purified antibody composition,
   wherein the ratio of glycosylation isomers relative to the total antibody in the purified antibody composition is reduced when compared to the crude antibody product.
(24) An antibody composition produced by the production method according to (22) or (23) above.
(25) A method for removing glycosylation isomers having sugar chains attached to sites other than the Fc region glycosylation consensus region from a crude anti-hDLK-1 antibody product, whose heavy chain has an amino acid sequence selected from SEQ ID Nos: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, said method comprising:
   loading the crude antibody product on a hydrophobic interaction chromatography media to allow an antibody having no sugar chains attached to sites other than the Fc region glycosylation consensus region to be adsorbed to the media;
   washing the media with a wash buffer; and
   treating the media with an eluent to thereby elute the antibody adsorbed to the media to obtain a purified antibody composition.
(26) An anti-hDLK-1 antibody composition, whose heavy chain has an amino acid sequence selected from SEQ ID Nos: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, wherein the antibody composition contains an antibody having no sugar chains attached to sites other than the Fc region glycosylation consensus region at a ratio of 95% or more relative to the total antibody.
(27) An anti-hDLK-1 antibody, whose heavy chain has an amino acid sequence selected from SEQ ID Nos: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, wherein the antibody has no sugar chains attached to sites other than the Fc region glycosylation consensus region.

### Effects of the Invention

The present invention enables the reduction or removal of glycosylation isomers having sugar chains attached to sites other than the glycosylation consensus region in an antibody.

As a first effect, the present invention enables the provision of an antibody composition for medical use with reduced content of glycosylation isomers. The thus purified high-purity antibody composition with reduced levels of glycosylation isomers can be formulated into pharmaceutical formulations with higher purity.

As a second effect, the present invention enables the provision of an antibody composition for medical use free from glycosylation isomers. The thus obtained antibody composition can be provided as a composition whose active pharmaceutical ingredient is of very high purity, i.e., as a pharmaceutical composition excellent in efficacy and safety.

In particular, the purified composition of anti-hDLK-1 antibody purified in the present invention is free from inactive impurities, i.e., glycosylation isomers, and therefore can be provided as a pharmaceutical composition excellent in efficacy and safety.

### Brief Description of the Drawings

Figure 1 is a graph showing an HIC-HPLC analysis pattern of the crude product obtained from the culture solution of cells producing the humanized anti-hDLK-1 monoclonal antibody.
Figure 2 is a photograph showing the results of SDS-PAGE analysis on Protein A-purified culture supernatants from CHO cells transiently expressing the humanized anti-hDLK-1 monoclonal antibody.
Figure 3 is a graph showing a separation profile of the antibody composition with the Capto Butyl hydrophobic interaction chromatography media.
Figure 4 includes a graph and table showing the results of purity analysis test by HIC-HPLC on the purified antibody composition separated with the Capto Butyl media. In the table, Peak 1 and Peak 2 each shows the ratio (%) of glycosylation isomer antibody (Peak 1 has a sugar chain linked to one CDR in the four polypeptide chains, Peak 2 has sugar chains linked to two CDRs in the four polypeptide chains), and Peak 3 shows the ratio (%) of antibody having a sugar chain linked only to the glycosylation consensus region.
Figure 5A is a graph showing a separation profile of the antibody composition with the Capto Butyl media.
Figure 5B is a graph showing a separation profile of the antibody composition with the Poros Benzyl Ultra media.
Figure 6 includes graphs showing the results analyzed by the design of experiment for control of the glycosylation isomer content by optimizing the conditions used for chromatography with the POROS Benzyl Ultra media.
Figure 7 is a diagram showing the sequence of chromatography steps in the purification process of the antibody composition.
Figure 8 is a graph showing the elution pattern, yield and purity obtained when varying the volumes of wash buffers in hydrophobic interaction chromatography (upper panel: under conditions where wash buffer 1 is 6 CV and wash buffer 2 is 5 CV; middle panel: under conditions where wash buffer 1 is 8 CV and wash buffer 2 is 5 CV; lower panel: under conditions where wash buffer 1 is 15 CV and wash buffer 2 is 5 CV).
Figure 9 shows the analysis data of HIC-HPLC analysis on components isolated from the crude antibody product by precise fractionation. Panel (a) shows the results of HIC-HPLC analysis on the crude antibody product before isolation. Panel (b) shows the results of HIC-HPLC analysis on the fraction obtained as a main peak during HIC-HPLC in (a). Panel (c) shows the results of HIC-HPLC analysis on the fraction obtained as a pre-peak during HIC-HPLC in (a).
Figure 10 is a graph showing the results evaluated for the ADCC activity of glycosylation isomers. The vertical axis shows the fluorescence intensity indicative of ADCC activity, and the horizontal axis shows the antibody concentration. Circles represent the results of the fraction obtained as a main peak in Figure 9 (b) (glycosylation isomer-free component), squares represent the results of the fraction obtained as a pre-peak in Figure 9 (c) (glycosylation isomer), and triangles represent the results of the crude antibody product before isolation in Figure 9 (a).

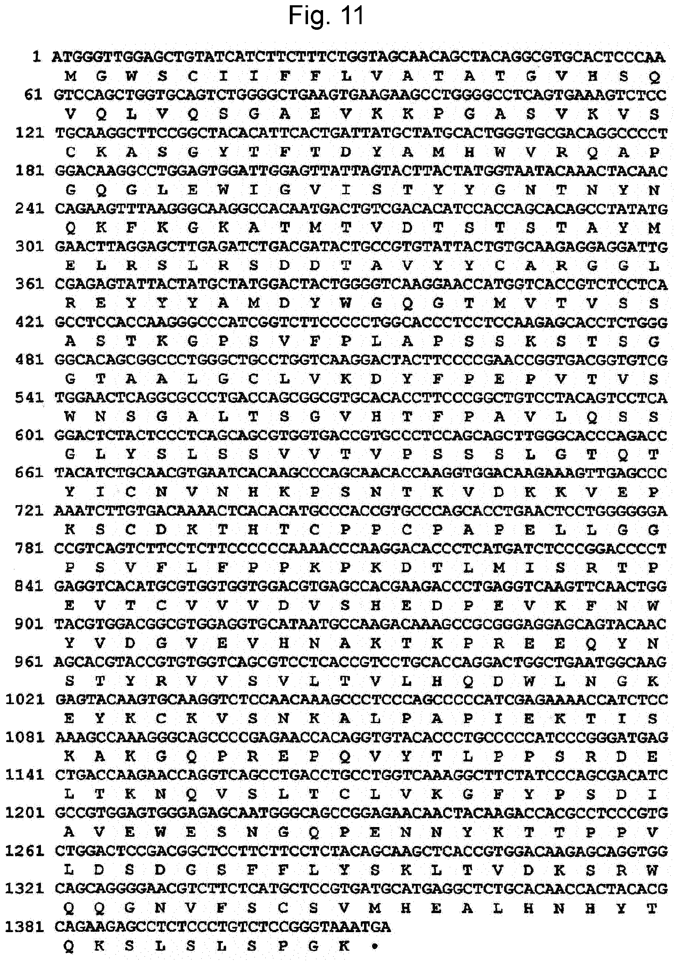

Figure 11 shows the nucleotide sequence (SEQ ID NO: 37) and amino acid sequence (SEQ ID NO: 38) of the coding region for the H chain (γ1 chain) of HuBA-1-3D-1. Amino acids are expressed in single-letter notation, and the position of the termination codon is indicated with "●" (black dot).

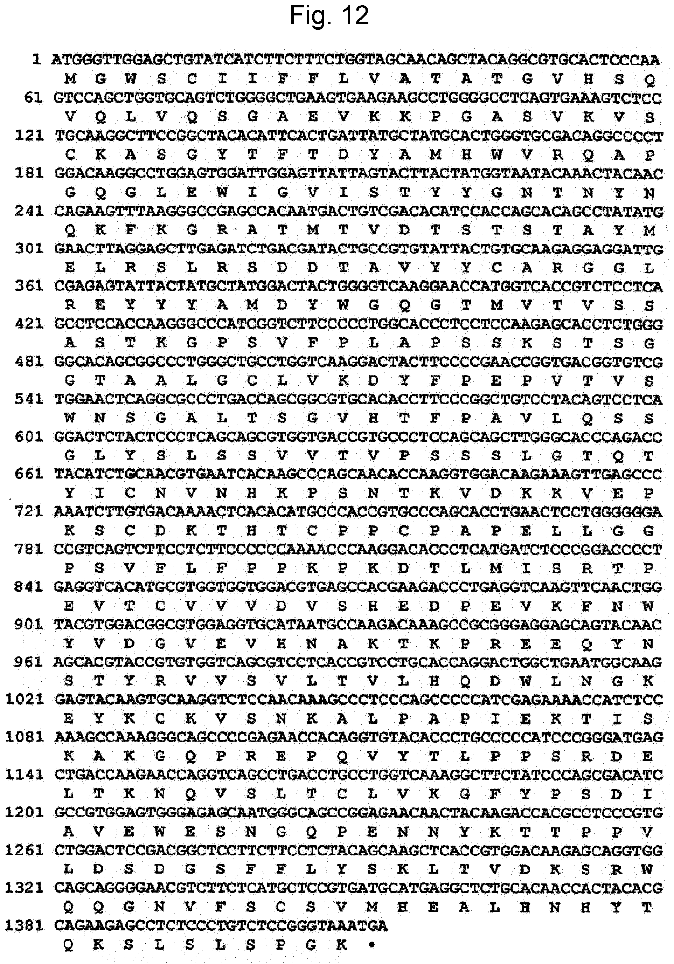

Figure 12 shows the nucleotide sequence (SEQ ID NO: 41) and amino acid sequence (SEQ ID NO: 42) of the coding region for the H chain (γ1 chain) of HuBA-1-3D-2. Amino acids are expressed in single-letter notation, and the position of the termination codon is indicated with "●" (black dot).

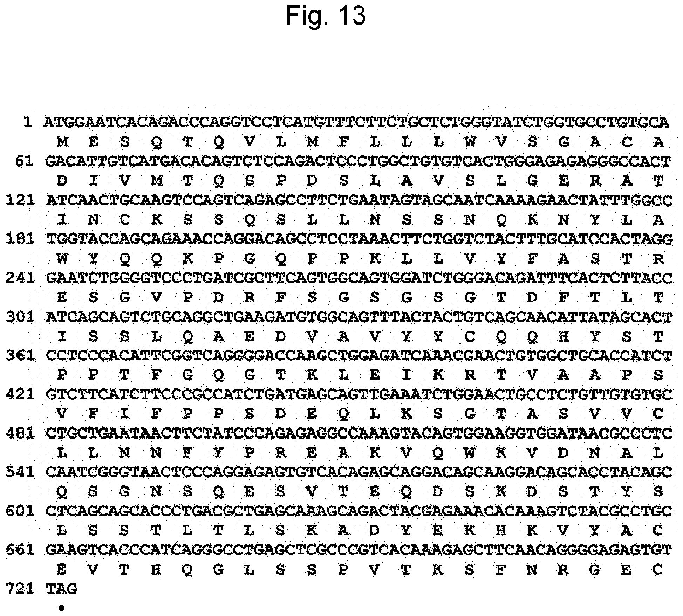

Figure 13 shows the nucleotide sequence (SEQ ID NO: 69) and amino acid sequence (SEQ ID NO: 70) of the coding region for the L chain (κ chain). In the figure, amino acids are expressed in single-letter notation, and the position of the termination codon is indicated with "●" (black dot).

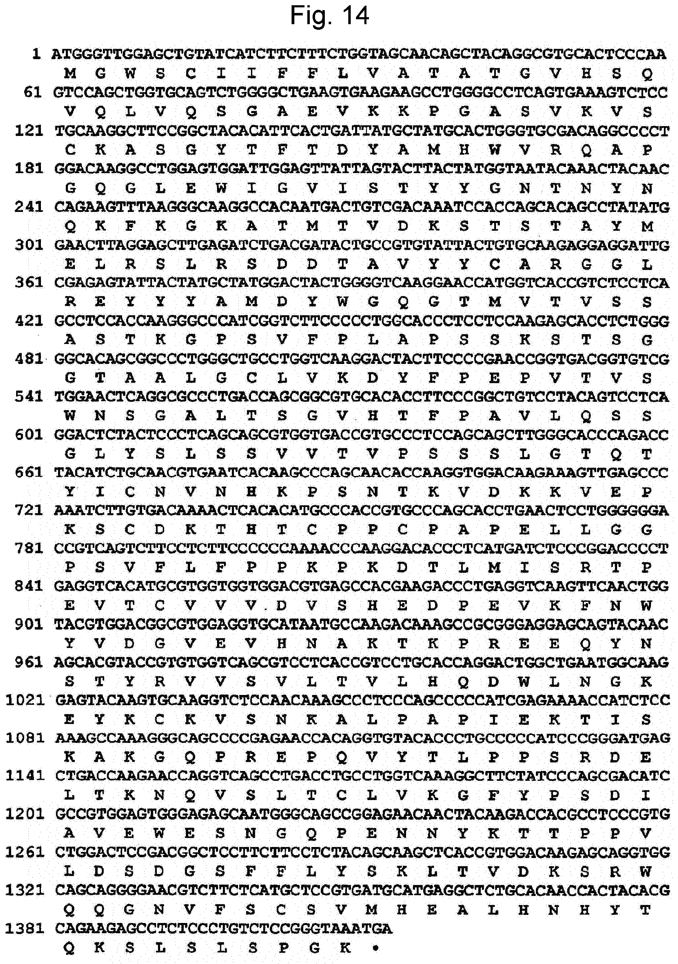

Figure 14 shows the nucleotide sequence (SEQ ID NO: 53) and amino acid sequence (SEQ ID NO: 54) of the coding region for the H chain (γ1 chain) of HuBA-1-3D-1 T73K. Amino acids are expressed in single-letter notation, and the position of the termination codon is indicated with "●" (black dot). In the deduced VH amino acid sequence (SEQ ID NO: 18) of the above HuBA-1-3D-1 T73K, the N-terminal peptide of 19 amino acids is a signal peptide. The cDNA nucleotide sequence of a mature VH peptide of HuBA-1-3D-1 T73K is shown in SEQ ID NO: 19, and its deduced amino acid sequence is shown in SEQ ID NO: 20.

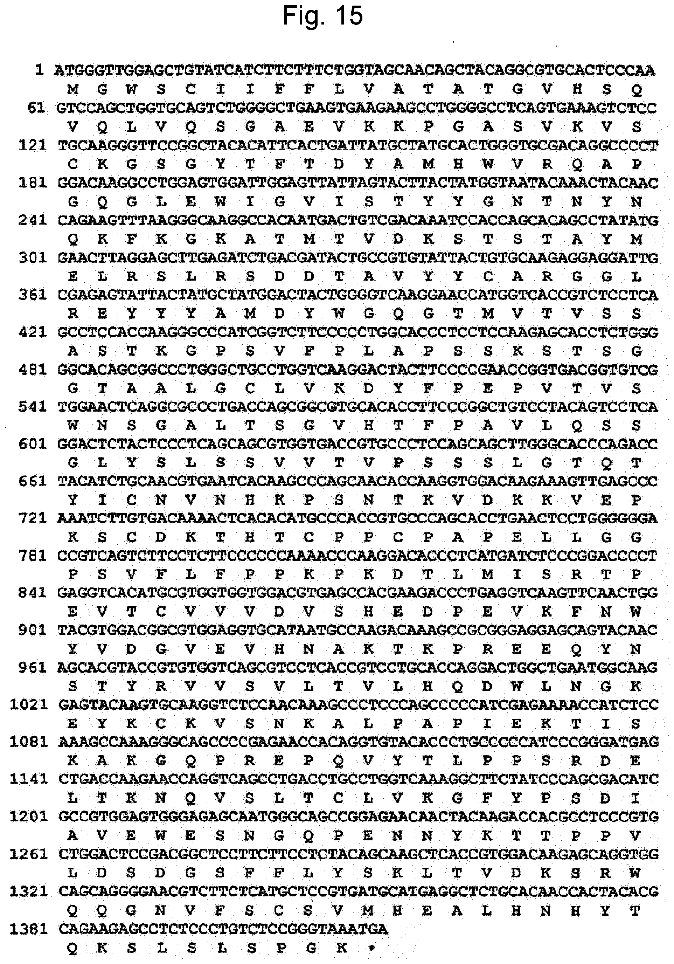

Figure 15 shows the nucleotide sequence (SEQ ID NO: 61) and amino acid sequence (SEQ ID NO: 62) of the coding region for the H chain (γ1 chain) of HuBA-1-3D-1 A24G/T73K. Amino acids are expressed in single-letter notation, and the position of the termination codon is indicated with "●" (black dot). In the deduced VH amino acid sequence (SEQ ID NO: 22) of the above HuBA-1-3D-1 A24G/T73K, the N-terminal peptide of 19 amino acids is a signal peptide. The cDNA nucleotide sequence of a mature VH peptide of HuBA-1-3D-1 A24G/T73K is shown in SEQ ID NO: 23, and its deduced amino acid sequence is shown in SEQ ID NO: 24.

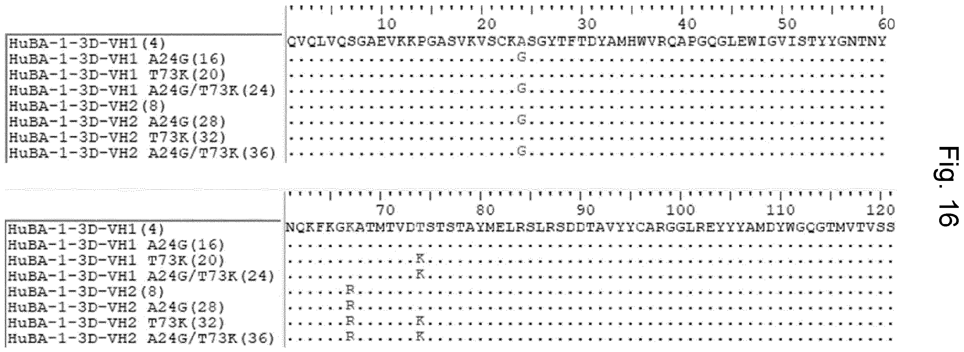

Figure 16 shows the amino acid sequence alignment of HuBA-1-3D-VH1, HuBA-1-3D-VH1 A24G, HuBA-1-3D-VH1 T73K, HuBA-1-3D-VH1 A24G/T73K, HuBA-1-3D-VH2, HuBA-1-3D-VH2 A24G, HuBA-1-3D-VH2 T73K and HuBA-1-3D-VH2 A24G/T73K, whose SEQ ID Nos in the Sequence Listing are given in parentheses.

### Description of Embodiments

The present invention will be described in more detail below. The scope of the present invention is not limited by the following descriptions, and any embodiments other than those illustrated below may also be carried out with appropriate modifications without departing from the spirit of the invention. It should be noted that this specification incorporates the specification of Japanese Patent Application No. 2021-079977 (filed on May 10, 2021) in its entirety, based on which the present application claims priority. Moreover, all publications cited herein, including prior art documents, patent gazettes and other patent documents, are incorporated herein by reference.

As used herein, the term "glycosylation consensus sequence" is intended to mean an amino acid sequence represented by Asn-X-Ser/Thr (wherein X is an amino acid other than Pro), regardless of the position of this sequence in an antibody and the position where Asn is present. As used herein, the term "Fc region glycosylation consensus region" or "glycosylation consensus region" refers to a glycosylation consensus sequence usually comprising Asn297 present in the antibody Fc portion (typically, Asn297-X-Ser/Thr (wherein X is an amino acid other than Pro)). A nucleotide sequence encoding such a glycosylation consensus sequence or glycosylation consensus region includes any sequences as long as they each encode this amino acid sequence.

As used herein, the term "glycosylation isomers" is intended to mean antibodies having sugar chains attached to amino acids other than those in the glycosylation consensus region. Sugar chains attached to amino acids other than those in the glycosylation consensus region are referred to as "non-consensus sugar chains." In glycosylation isomers, regions to which non-consensus sugar chains are linked include an Fab region, an antigen-binding Fv (variable) region, a complementarity determining region (CDR), an Fc region except for the glycosylation consensus region, and a fusion sequence portion in a fusion antibody, typically a CDR region. As to the sugar chain linkage mode of non-consensus sugar chains to amino acids other than those in the glycosylation consensus region, they may be N-linked sugar chains to the glycosylation consensus sequence Asn-X-Ser/Thr (wherein X is an amino acid other than Pro) or O-linked sugar chains to Ser or Thr.

The structure of an antibody molecule is generally a heterotetramer and is formed from two sets of two identical polypeptide chains joined together. Thus, linkage sites for non-consensus sugar chains are present at multiples of 2 per antibody molecule in theory. A crude antibody product to be purified in the present invention may contain antibodies having one or more non-consensus sugar chains. The number of non-consensus sugar chains linked per antibody molecule may be 1 or more, 2 or more, 3 or more, 4 or more, 5 or more, 6 or more, 7 or more, 8 or more, 9 or more, or 10 or more. Moreover, since an antibody is composed of four polypeptide chains (usually two heavy chains and two light chains), the number of non-consensus sugar chains linked to any one of these polypeptide chains may be 1 or more, 2 or more, 3 or more, 4 or more, 5 or more, 6 or more, 7 or more, 8 or more, 9 or more, or 10 or more. The ratio of sugar chains linked to regions to which non-consensus sugar chains are linkable in a crude antibody product, i.e., the ratio of glycosylation isomers present in a crude antibody product may be 0.1% or more and 200% or less per antibody molecule, relative to the case where non-consensus sugar chains are completely linked to one of the "combinations of heavy and light chains" in one antibody molecule or a structure equivalent thereto, which is set to 100%. This ratio is usually 1% or more and 50% or less in a crude antibody product in need of applying the method of the present invention. 200% intended here means that non-consensus sugar chains are linked to both of the pairing "combinations of heavy and light chains" (i.e., two sites) in one antibody molecule.

As used herein, the term "antibody" includes not only a full-length antibody, but also an antibody fragment, and a fusion product of a full-length antibody or an antibody fragment with another substance. Examples include a mouse antibody, a mouse-human chimeric antibody, a humanized antibody, a human antibody, and their amino acid variants, addition variants, deletion variants, substitution variants and sugar chain variants, etc. The immunoglobulin class of an antibody is not limited in any way, and may be any of the immunoglobulin classes (isotypes) IgG, IgM, IgA, IgE, IgD and IgY, preferably IgG. Moreover, in the case of the IgG class, the antibody of the present invention may be of any subclass (IgG1, IgG2, IgG3 or IgG4). An antibody fragment is preferably an antigen-binding fragment, including F(ab')₂, Fab', Fab, Fabs, single-chain Fv (hereinafter referred to as "scFv"), (tandem) bispecific single-chain Fv (sc(Fv)₂), single-chain triple body, nanobody, divalent V_{H}H, pentavalent V_{H}H, minibody, (double-chain) diabody, tandem diabody, bispecific tribody, bispecific bibody, dual affinity retargeting molecule (DART), triabody (or tribody), tetrabody (or [sc(Fv)₂]₂, or (scFv-SA)₄), disulfide-stabilized Fv (hereinafter referred to as "dsFv"), compact IgG, heavy chain antibody, or polymers thereof. A fusion product of an antibody fragment with another substance may be exemplified by a fusion protein, particularly an Fc fusion protein.

In particular, the antibody intended herein means an anti-hDLK-1 antibody whose heavy chain has an amino acid sequence selected from SEQ ID Nos: 2, 4, 6, 8, 14, 16, 18, 20, 22, 24, 26, 28, 30, 32, 34 and 36, and whose light chain has the amino acid sequence shown in SEQ ID NO: 10 or 12. It is preferably an anti-hDLK-1 antibody whose heavy chain has an amino acid sequence selected from SEQ ID Nos: 4, 8, 16, 20, 24, 28, 32 and 36, and whose light chain has the amino acid sequence shown in SEQ ID NO: 12.

An antibody composition containing glycosylation isomers to be purified is herein referred to as a "crude antibody product." Any crude antibody product may be used as long as it contains glycosylation isomers. Examples of a crude antibody product include a biological composition (e.g., plasma) or a treated product thereof, and a culture solution (which may be a culture supernatant; the same applies hereinafter) of antibody gene-transfected transformed cells or a treated product thereof. Such a biological composition may be exemplified by a composition comprising antibodies obtained from a transgenic non-human animal or a plant, etc. Transformed cells are not limited in any way as long as they allow glycosylation, and specific examples include cell lines of animal, plant or yeast origin, which have the property of allowing glycosylation, and more specific examples include Chinese hamster ovary cells (CHO cells), mouse myeloma cells (NS0 cells, SP2/0 cells), rat myeloma cells (YB2/0 cells, IR983F cells), Syrian hamster kidney-derived BHK cells, human fetal kidney-derived 293 cells, human myeloma cells (Namalwa cells), embryonic stem cells, or antibody gene-transfected fertilized eggs, etc. As to the above cell lines, it is also possible to use various subspecies derived from primary immortalized cell lines. For example, in the case of CHO cells, it is possible to use CHO K1, CHO DG44 and CHO S cell lines, and their derived cell lines, etc. (Palsson et al., Nature Biotechnology 31(8), 759-765, 2013). When these cells are cultured in a medium suitable for protein production, a crude antibody product may be obtained as a culture solution. As to the medium used for this purpose, examples include a serum-containing medium, a medium containing no animal-derived component such as serum albumin or serum fraction, a serum-free medium and a protein-free medium, and preferred for use is a serum-free medium, a medium containing no animal-derived material, a protein-free medium, or a completely chemical synthetic medium.

Further, as to the above crude antibody product, it is also possible to use a biological composition or a culture solution treated by filtration, salting-out, one or more chromatography techniques, pH adjustment, buffer replacement, concentration, dilution, etc., or an intermediate composition derived from the biological composition or culture solution during purification or other operations. As an intermediate composition derived during purification, it is possible to use even a solution obtained after any unit operations required to construct the production process of antibody drugs. For example, it is desired to use a composition obtained after Protein A affinity chromatography, after cation exchange chromatography, after anion exchange chromatography, after buffer replacement, after low pH treatment, or after filtration, etc.

In the case of N-linked sugar chains, the possible presence of glycosylation isomers can be estimated from the amino acid sequence or gene sequence of the antibody. Moreover, regardless of the linkage mode of sugar chains, the positions for sugar chain linkages can be estimated by peptide mapping and mass spectrometry on the antibody. More conveniently, a peptide-N-glycosidase (PNGase)-treated antibody and a non-treated antibody may be compared by SDS polyacrylamide gel electrophoresis (SDS-PAGE), whereby sugar chain linkages can be observed as changes in protein electrophoretic bands.

Although the type of antibody is as shown above, this method is suitable as a purification method in the production of antibodies for use as antibody drugs including therapeutic, diagnostic or prophylactic antibodies. Antibody drugs are required to have a consistency in the content of antibody isomers contained therein, and are required to minimize product-related impurities (the ICH Q6B guideline). Examples of a therapeutic or prophylactic antibody include an antibody neutralizing the activity of a ligand through binding to the ligand, an antibody neutralizing the binding of a ligand through binding to its receptor on the cell surface, and an antibody exerting cytotoxic activity on cells themselves through binding to their cell surface. Examples of a diagnostic antibody include an antibody binding to a ligand or a receptor on the cell surface. The cytotoxic activity on cells may be exemplified by antibody-dependent cellular cytotoxicity, complement-dependent cytotoxicity, antibody-dependent cellular phagocytosis activity, etc. Moreover, the technique of the present invention may also be used for antibody derivatives as long as they have sugar chains, as exemplified by chemically modified antibodies (e.g., antibody-drug conjugates, radioisotope-labelled antibodies), fusion antibodies with cytokines, etc., and multi-specific antibodies (Nature, 580(16), 330-338 (2020)), etc.

In more detail, this method can be used in the purification of antibodies against protein antigens (preferably protein antigens of human origin), including CD3, EGF receptor, CD20, RS virus, TNFα, CD25, IL-6 receptor, CD33, VEGF, IgE, complement C5, IL-12, IL-23, IL-1β, RANKL, CCR4, HER2, CD30, IL-5, IL-5 receptor, α4 integrin, α4β7 integrin, PD-1, CD52, IL-17, IL-17A, IL-17 receptor, CTLA-4, PCSK9, SLAMF7, BlyS, CD38, PD-L1, IL-4α receptor, CD22, CD23, factor Ixa, factor X, CD19, sclerostin, DLK-1, etc. Examples of a fusion protein include a soluble TNF receptor Fc fusion protein, a CTLA4-modified Fc fusion protein, a Fc-TPOR agonist peptide fusion protein, a VEGF receptor-Fc fusion protein, etc. This method can be used in the purification of any of these antibodies or fusion proteins, etc.

One of the most desired cases is the purification of an anti-DLK-1 antibody. In more detail, it is a humanized anti-human DLK-1 antibody as appears in Patent Document 4 (WO2014/054820) given above, as exemplified by an antibody comprising a heavy chain having an amino acid sequence selected from SEQ ID Nos: 2, 4, 6, 8, 14, 16, 18, 20, 22, 24, 26, 28, 30, 32, 34 and 36 (particularly a heavy chain having any of these amino acid sequences as a variable region; the same applies hereinafter in this paragraph), and a light chain having the amino acid sequence shown in SEQ ID NO: 10 or 12 (particularly a light chain having any of these amino acid sequences as a variable region; the same applies hereinafter in this paragraph), and preferred is an antibody comprising a heavy chain having an amino acid sequence selected from SEQ ID NO: 4, 8, 16, 20, 24, 28, 32 and 36, and a light chain having the amino acid sequence shown in SEQ ID NO: 12. Such a humanized anti-human DLK-1 monoclonal antibody has a glycosylation consensus sequence in the variable region of its light chain.

Tables 1 and 2 below contain the full-length sequence of the anti-human DLK-1 antibody shown in Patent Document 4 (WO2014/054820) given above (each underlined section represents a signal sequence, and the underlined sequence is not contained in a mature protein). For example, in this antibody, N in the boxed NSS sequence serves as a glycosylation consensus sequence. For this reason, when animal cells or others are engineered to express this gene, there is a possibility that these cells will produce a sugar chain composition containing glycosylation isomers. Likewise, DLK-1 antibodies having sequences similar to the sequence of this anti-DLK-1 antibody also have the same possibility. This method is useful as a method for removing or reducing glycosylation isomers from such a crude antibody product containing glycosylation isomers. It should be noted that the amino acid sequences of the H chain of HuBA-1-3D-1, the H chain of HuBA-1-3D-2, the H chain of HuBA-1-3D-1 A24G, the H chain of HuBA-1-3D-2 A24G, the H chain of HuBA-1-3D-1 T73K, the H chain of HuBA-1-3D-2 T73K, the H chain of HuBA-1-3D-1 A24G/T73K, the H chain of HuBA-1-3D-2 A24G/T73K, and the L chain of HuBA-1-3D given in the tables below are shown in SEQ ID Nos: 38, 42, 46, 50, 54, 58, 62, 66 and 70, respectively, in this order. Moreover, the amino acid sequences of mature proteins produced upon removal of signal sequences from the above sequences are shown in SEQ ID Nos: 40, 44, 48, 52, 56, 60, 64, 68 and 72, respectively, in this order. As used herein and elsewhere, the term "H chain" refers to a heavy chain, and the term "L chain" refers to a light chain.

In HuBA-1-3D VH1 and HuBA-1-3D VH2, the amino acid sequence of CDR1 is "DYAMH" (SEQ ID NO: 73), the amino acid sequence of CDR2 is "VISTYYGNTNYNQKFKG" (SEQ ID NO: 74), and the amino acid sequence of CDR3 is "GGLREYYYAMDY" (SEQ ID NO: 75). Likewise, in HuBA-1-3D VL, the amino acid sequence of CDR1 is "KSSQSLLNSSNQKNYLA" (SEQ ID NO: 76), the amino acid sequence of CDR2 is "FASTRES" (SEQ ID NO: 77), and the amino acid sequence of CDR3 is "QQHYSTPPT" (SEQ ID NO: 78). The antibody of the present invention may be an antibody having all or some of these CDRs. It should be noted that these CDR sequences were according to the definition of Kabat et al. (Sequences of Proteins of Immunological Interests, Fifth edition, NIH Publication No.91-3242, U.S. Department of Health and Human Services, 1991). As used herein and elsewhere, the term "VH" refers to a heavy chain variable region, and the term "VL" refers to a light chain variable region.

**[Table 1]**

| |
|---|
| **HuBA-1-3D-1 Heavy chain** |
| MGWSCIIFFLVATATGVHSQVQLVQSGAEVKKPGASVKVSCKASGYTFTDYAMHWVRQAP GQGLEWIGVISTYYGNTNYNQKFKGKATMTVDTSTSTAYMELRSLRSDDTAVYYCARGGL REYYYAMDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| **HuBA-1-3D-1 A24G Heavy chain** |
| MGWSCIIFFLVATATGVHSQVQLVQSGAEVKKPGASVKVSCKGSGYTFTDYAMHWVRQAP GQGLEWIGVISTYYGNTNYNQKFKGKATMTVDTSTSTAYMELRSLRSDDTAVYYCARGGL REYYYAMDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| **HuBA-1-3D-1 T73K Heavy chain** |
| MGWSCIIFFLVATATGVHSQVQLVQSGAEVKKPGASVKVSCKASGYTFTDYAMHWVRQAP GQGLEWIGVISTYYGNTNYNQKFKGKATMTVDKSTSTAYMELRSLRSDDTAVYYCARGGL REYYYAMDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| **HuBA-1-3D-1 A24G/T73K Heavy chain** |
| MGWSCIIFFLVATATGVHSQVQLVQSGAEVKKPGASVKVSCKGSGYTFTDYAMHWVRQAP GQGLEWIGVISTYYGNTNYNQKFKGKATMTVDKSTSTAYMELRSLRSDDTAVYYCARGGL REYYYAMDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |

**[Table 2]**

| |
|---|
| **HuBA-1-3D-2 Heavy chain** |
| MGWSCIIFFLVATATGVHSQVQLVQSGAEVKKPGASVKVSCKASGYTFTDYAMHWVRQAP GQGLEWIGVISTYYGNTNYNQKFKGRATMTVDTSTSTAYMELRSLRSDDTAVYYCARGGL REYYYAMDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| **HuBA-1-3D-2 A24G Heavy chain** |
| MGWSCIIFFLVATATGVHSQVQLVQSGAEVKKPGASVKVSCKGSGYTFTDYAMHWVRQAP GQGLEWIGVISTYYGNTNYNQKFKGRATMTVDTSTSTAYMELRSLRSDDTAVYYCARGGL REYYYAMDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| **HuBA-1-3D-2 T73K Heavy chain** |
| MGWSCIIFFLVATATGVHSQVQLVQSGAEVKKPGASVKVSCKASGYTFTDYAMHWVRQAP GQGLEWIGVISTYYGNTNYNQKFKGRATMTVDKSTSTAYMELRSLRSDDTAVYYCARGGL REYYYAMDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| **HuBA-1-3D-2 A24G/T73K Heavy chain** |
| MGWSCIIFFLVATATGVHSQVQLVQSGAEVKKPGASVKVSCKGSGYTFTDYAMHWVRQAP GQGLEWIGVISTYYGNTNYNQKFKGRATMTVDKSTSTAYMELRSLRSDDTAVYYCARGGL REYYYAMDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| **HuBA-VH1-3D LC Light chain** |
| MESQTQVLMFLLLWVSGACADIVMTQSPDSLAVSLGERATINCKSSQSLLNSSNQKNYLA WYQQKPGQPPKLLVYFASTRESGVPDRFSGSGSGTDFTLTISSLQAEDVAVYYCQQHYST PPTFGQGTKLEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVTKSFNRGEC |

An antibody composition whose glycosylation isomer content is reduced (herein referred to as a "purified antibody composition") when compared to a crude antibody product can be obtained when the crude antibody product containing glycosylation isomers is used as a starting material and subjected to conventional chromatography. Accordingly, the present invention relates to a purification method for a crude antibody product, said method comprising: loading the crude antibody product on conventional chromatography to allow an antibody having no sugar chains attached to sites other than the Fc region glycosylation consensus region to be adsorbed to the media; and treating the media with an eluent to thereby elute the antibody adsorbed to the media to obtain a purified antibody composition, wherein the content of glycosylation isomers having sugar chains attached to sites other than the Fc region glycosylation consensus region in the resulting purified antibody composition is reduced when compared to the crude antibody product before being subjected to purification.

As a media for use in conventional chromatography, it is possible to use a hydrophobic interaction chromatography media, a hydrophobic chromatography media, or a mixed-mode chromatography media (also referred to as a multi-mode chromatography media, preferably a mixed-mode chromatography media having the nature of hydrophobic chromatography). For use as a hydrophobic interaction chromatography media, a base substrate may be attached with hydrophobic functional groups such as a methyl group, an ethyl group, a propyl group, a butyl group, an octyl group, a hexyl group, a propylene glycol group, a phenyl group, an alkylphenyl group, a benzyl group, and an alkylbenzyl group, etc. For use as a mixed-mode chromatography media, the above hydrophobic functional groups and ion-exchangeable functional groups may be mixed in any ratio. For example, N-benzyl-N-methylethanolamine and so on may be used as functional groups. Representative examples of cation-exchangeable functional groups include CM (carboxymethyl, -O-CH₂-COOH), SP (sulfopropyl, -O-C₃H₆-SO₃H) and so on, while representative examples of anion-exchangeable functional groups include DEAE (diethylaminoethy, -O-C₂H₄-N-(C₂H₅)₂), QAE (quaternized aminoethyl or diethyl-(2-hydroxypropyl)-aminoethyl, -O-C₂H₄-N-(C₂H₅)₂(CH₂-CH(OH)-CH₂)) and so on, and these functional groups may be used. Examples of a substrate for the media include cellulose, Sephadex, crosslinked agarose, polyacrylamide, methacrylate and various synthetic polymers. The media may or may not be porous, and either may be used. Another form of a mixed-mode chromatography media may be exemplified by those having functional groups such as calcium phosphate, like hydroxyapatite (Ca10(PO₄)₆(OH)₂) or fluoroapatite (Ca₁₀(PO₄)₆F₂).

A chromatography media may be obtained as a commercially available product and used. Specific examples include media such as Butyl-Sepharose^{®} 4 Fast Flow (average particle size: 90 µm), Butyl-S Sepharose 6 Fast Flow (average particle size: 90 µm), Octyl Sepharose^{®} 4 Fast Flow (average particle size: 90 µm), Phenyl Sepharose^{®} 6 Fast Flow (high sub) (average particle size: 90 µm), Phenyl Sepharose^{®} 6 Fast Flow (low sub) (average particle size: 90 µm), Butyl Sepharose^{®} High Performance (average particle size: 90 µm), Phenyl Sepharose High Performance (average particle size: 90 µm), SOURCE 15ETH (average particle size: 15 µm), SOURCE 15ISO (average particle size: 15 µm), SOURCE 15PHE (average particle size: 15 µm), Capto Phenyl (High Sub) (average particle size: 90 µm), Capto Butyl (average particle size: 90 µm), Capto Octyl (average particle size: 90 µm), Capto Phenyl ImpRes (average particle size: 36-44 µm), Capto Butyl ImpRes (average particle size: 36-44 µm), Capto adhere (average particle size: 90 µm), Capto adhere ImpRes (average particle size: 36-44 µm), Capto MMC (average particle size: 90 µm), Capto MMC ImpRes (average particle size: 36-44 µm), Capto Core 700 (average particle size: 90 µm), ReadyToProcess Adsorber Phenyl (which are all products of Cytiva, UK), TOYOPEARL^{®} Butyl-600 (average particle size: 40-90 µm), TOYOPEARL^{®} Phenyl-600 (average particle size: 40-90 µm), TOYOPEARL^{®} PPG-600 (average particle size: 40-90 µm), TOYOPEARL^{®} Butyl-650 (average particle size: 40-90 µm), TOYOPEARL^{®} Phenyl-650 (average particle size: 40-90 µm), TOYOPEARL^{®} SuperButyl-550 (average particle size: 40-90 µm), TOYOPEARL^{®} Hexyl-650 (average particle size: 50-150 µm), TOYOPEARL^{®} Ether-650 (average particle size: 40-90 µm) (which are all products of Tosoh Corporation, Japan), POROS Ethyl (average particle size: 50 µm), POROS Benzyl (average particle size: 50 µm), POROS Benzyl Ultra (average particle size: 50 µm) (which are all products of Thermo Fisher), Macro-Prep t-Butyl HIC (average particle size: 50 µm), Macro-Prep Methyl HIC (average particle size: 50 µm), ceramic hydroxyapatite (average particle size: 20-80 µm), ceramic fluoroapatite (average particle size: 20-80 µm), biogel HT (average particle size: 20-80 µm) (which are all products of Bio-Rad Laboratories, Inc), QMA Spherosil (average particle size: 50 µm), Methyl Ceramic Hyper D (average particle size: 50 µm) (which are all products of Pall Corporation), Fractogel EMD Phenyl (S) (average particle size: 20-90 µm), Fractogel EMD Propyl (S) (average particle size: 20-90 µm) (which are all products of Merck & Co., Inc.), Cellufine MAX Phenyl (average particle size: 40-130 µm), Cellufine MAX Phenyl LS (average particle size: 40-130 µm), Cellufine MAX Butyl (average particle size: 40-130 µm) (which are all products of JNC), butylated Chitopearl, phenylated Chitopearl (which are all products of Fujibo Holdings, Inc., Japan), etc. From among these media, a more suitable media is selected and used. More preferred are media such as Capto Butyl, POROS Benzyl Ultra, POROS Butyl Ultra, etc.

The average particle size of the chromatography media used for purification purposes in the present invention may be set to 15 µm or more, 20 µm or more, 30 µm or more, or 40 µm or more. Moreover, the volume of a crude antibody product which can be treated with the chromatography media may be set to 100 mL or more, 1 L or more, 10 L or more, or 100 L or more. As a chromatography column to be filled with this media, it is possible to use a chromatography column whose volume is 100 mL to around 1,000 L (diameter: 5 cm to around 2 m). The volume of a crude antibody product provided for chromatographic purification is at least 1 L or more, desirably 10 L or more, more desirably 100 L or more, even more desirably 500 L or more, and up to around 20,000 L. Due to the necessity to treat such a large volume of a crude antibody product, the linear flow rate in chromatography is 1,000 cm/hr or less, and desirably 500 cm/hr or less. The amount of antibody provided for purification is 10 g or more, desirably 100 g or more, more desirably 1 kg or more, and even more desirably 10 kg or more, calculated as the amount of protein. Glycosylation isomers to be removed or reduced by this technique are contained in the first half fraction of antibody eluted by chromatography, and the desired antibody of interest is eluted into the second half fraction, whereby the glycosylation isomers are fractionated and removed.

Conditions under which a crude antibody product containing glycosylation isomers is loaded on conventional chromatography should at least be sufficient to allow a component of interest, i.e., an antibody having a sugar chain attached only to the glycosylation consensus region (non-glycosylation isomer antibody) to be adsorbed to the chromatography media. Antibody adsorption is caused by interaction between the antibody and the chromatography media based on the degree of hydrophobicity-hydrophilicity. As a buffer, it is possible to use a buffer commonly used in hydrophobic interaction chromatography, hydrophobic chromatography, or mixed-mode chromatography. Any buffer may be used as long as the antibody is stable under chromatography conditions, and examples include phosphate buffer, acetate buffer, citrate buffer, Tris buffer, glycine buffer, borate buffer, tartrate buffer, MES buffer, HEPES buffer, MOPS buffer, amino acid buffer, and mixed buffers thereof. The concentration of these buffers may be selected freely within the commonly used range of around 0.1 mM to 300 mM. The buffer pH may be selected freely within the range of pH 4 to 8, but it is preferably pH 4 to 6.

To these buffers, a salt such as sodium sulfate, ammonium sulfate, sodium chloride or sodium citrate may optionally be added in an appropriate amount not to cause antibody precipitation, thereby enhancing antibody interaction with the above chromatography media and thus allowing antibody adsorption to the chromatography media. The above salt may be selected freely from one or more candidates. As to the salt concentration, antibodies are present stably in the range of 300 mM to 2 M salt, and the concentration used is required to allow sufficient antibody adsorption to the chromatography media. The salt concentration is preferably around 1 M, and a lower salt concentration allowing antibody adsorption is preferred for this purpose. The amount of antibody which can be adsorbed per unit amount of the chromatography media will widely vary depending on the type of the chromatography media and the conditions of the buffer, but 10 mg or more of antibody per mg of the media, preferably 20 mg or more of antibody per mg of the media can be adsorbed. The chromatography may be operated at any temperature in the range of 0°C to 40°C. The chromatography is desirably operated at room temperature, and more desirably operated under conditions where the temperature is controlled.

After the crude antibody product containing glycosylation isomers is loaded on chromatography under the conditions mentioned above, separation and purification are conducted by elution. The separation of glycosylation isomers by chromatography may be accomplished as follows: after antibody adsorption to the chromatography media the buffer to be passed through the column is changed to reduce the salt concentration, increase the pH, reduce the conductivity, or combinations thereof, in a stepwise fashion, in a continuous fashion, or combinations thereof. After antibody adsorption to the chromatography media, glycosylation isomers may first be eluted as a major faction, followed by eluting an antibody having a sugar chain attached only to the glycosylation consensus region (non-glycosylation isomer antibody) to thereby prepare a purified antibody composition of interest. The term "elution" means that an antibody component bound to a chromatography media through hydrophobic interaction, etc., is treated to weaken its binding to the media, and the antibody component is released from the chromatography media. The conditions required to elute glycosylation isomers are buffer conditions where the interaction between the glycosylation isomers and the chromatography media is sufficiently weaker than the interaction between the non-glycosylation isomer antibody and the chromatography media. When the chromatography media is washed with a sufficient volume of the buffer under these conditions, only the glycosylation isomers can be eluted and removed while ensuring that only the non-glycosylation isomer antibody, which is an antibody of interest, remains adsorbed to the chromatography media.

Any buffer may be used to elute and remove (wash) the glycosylation isomers, as long as it has a 10 mM or more difference in salt concentration and/or a 0.2 Unit or more difference in pH from the buffer used to elute the non-glycosylation isomer antibody. After the non-glycosylation isomer antibody is adsorbed to the chromatography media at a high salt concentration (usually within the range of 300 mM to 2 M), the salt concentration of the wash buffer used to wash the chromatography media may be set to be equal to or higher than the salt concentration for elution. For example, the salt concentration of the wash buffer may be equal to the salt concentration required for antibody elution or may range from equal to 1 M higher than the salt concentration required for antibody elution, or may range from equal to 0.5 M higher than the salt concentration required for antibody elution, or may be 10 mM or more higher than the salt concentration required for antibody elution, for example, may be set to 0.5 M or more or 1.0 M or more. The pH of the wash buffer may be set to be equal to or up to 2 Units higher than the pH for antibody adsorption, or may be set to be equal to or up to 2 Units lower than the pH for antibody elution. For example, the pH of the wash buffer may be 0.2 Units lower than the pH of the eluent. The pH of the wash buffer may be in the range of pH 4 to 8. Moreover, the passing volume of the buffer (wash buffer) required to elute and remove the glycosylation isomers may be set to 2 column volumes (CV) or more, 3 CV or more, 4 CV or more, 5 CV or more, 10 CV or more, 15 CV or more, or 20 CV or more, relative to the chromatography column volume, or may be set to 5 to 20 CV, 5 to 15 CV, 5 to 10 CV, or 10 to 20 CV, relative to the chromatography column volume.

During the process from loading to washing, the pH and/or salt concentration of the crude antibody product may be changed in a stepwise fashion (e.g., one or more steps, two or more steps, three or more steps, several steps) or in a continuous fashion. The pH of the crude antibody product during the process from loading to washing is generally within the range of 4 to 6.

The conditions of the above salt concentration, pH and/or wash buffer volume may be determined as appropriate depending on the content of glycosylation isomers in the crude antibody product used as a starting material, and the properties of antibody per se including the isoelectric point and amino acid sequence, etc.

The chromatography-based method for separation of glycosylation isomers intended herein may comprise allowing the glycosylation isomers to flow through the chromatography media, and then eluting an antibody having a sugar chain attached only to the glycosylation consensus region, whereby a purified antibody composition containing the non-glycosylation isomer antibody with high purity can be prepared. The phrase "flow through" means that when a crude antibody product is loaded on a column filled with a chromatography media, glycosylation isomers are eluted out from the column without being adsorbed to the chromatography media. In this case, the glycosylation isomers in the crude antibody product may weakly interact with the chromatography media, but components containing these unwanted glycosylation isomers can be eliminated from the column by passing an equilibration buffer (desirably one or more column volumes) in a continuous or intermittent manner. As a buffer used to allow the glycosylation isomers to specifically flow through the column, a buffer is selected such that the interaction between the glycosylation isomers and the chromatography media is sufficiently weaker than the interaction between the antibody having a sugar chain linked only to the glycosylation consensus region and the chromatography media. When a sufficient volume of the buffer is used to allow only the glycosylation isomers to flow through from the chromatography media, only the antibody of interest can be adsorbed to the chromatography media. Then, a buffer whose salt concentration and other conditions differ from those of the above buffer is used to elute the bound antibody from the chromatography media, thereby preparing the desired purified antibody composition.

As a buffer used to allow only the glycosylation isomers to flow through the column, any buffer may be used as a wash buffer for flow through purposes as long as it has a 10 mM or more difference in salt concentration (i.e., a salt concentration of not less than 10 mM or higher) and/or a 0.2 Unit or more difference in pH (i.e., a pH of not less than 0.2 or lower) when compared to the eluent used to elute the antibody having a sugar chain linked only to the glycosylation consensus region. The salt concentration of the buffer may be set to be equal to or up to 1 M higher (preferably up to 0.5 M higher) than the salt concentration required to elute the non-glycosylation isomer antibody from the media. The salt concentration in this case is usually 100% or more relative to the salt concentration required for antibody elution. Alternatively, the column is washed with a wash buffer whose pH is equal to or up to 2 Units higher than the pH for antibody adsorption or with a wash buffer whose pH is equal to or up to 2 Units lower than the pH for antibody elution. The pH or/and salt concentration may be changed in a single step or several steps, or may be changed in a continuous fashion. Moreover, the salt concentration or pH of the buffer is usually adjusted within an appropriate range before the crude antibody product serving as a starting material is applied onto the chromatography media. As to the passing volume of the buffer (wash buffer) required to allow the glycosylation isomers to flow through the column, conditions are selected such that the passing volume is twice or more, desirably 5 times or more of the volume of the chromatography media. The passing volume of the wash buffer is more desirably 10 to 20 times or more of the volume of the chromatography media. The above salt concentration, pH or/and wash buffer volume may be determined as appropriate depending on the content of glycosylation isomers in the crude antibody product used as a starting material.

Since the amount of antibody in the crude antibody product loaded on the chromatography media, i.e., the protein load on the chromatography media affects the resolution of glycosylation isomers, the protein load per unit volume of the chromatography media is determined so as to achieve the desired yield and purity (glycosylation isomer content) in the resulting purified antibody composition. In general, there is a limit on the amount of protein which can be adsorbed to the media during chromatography operation, so that the chromatography operation is controlled on the basis of parameters such as a maximum dynamic binding capacity (DBC). In general, a protein load below the maximum dynamic binding capacity provides good recovery and resolution of protein, and the desired separation effect of chromatography can be expected. Moreover, in the method of the present invention, a protein load above a certain amount is preferred to avoid the adsorption of unwanted glycosylation isomers, and protein is loaded in an amount which is at least 20 mg or more, 25 mg or more, 30 mg or more, or 35 mg or more, per unit amount (1 g) of the chromatography media or per unit volume (1 mL) of the chromatography media, and is equal to or less than the maximum dynamic binding capacity, whereby the glycosylation isomers are separated and removed. The protein load can be determined depending on the content of glycosylation isomers in the crude antibody product to be purified. Namely, if the content of glycosylation isomers is high in the crude antibody product before chromatographic purification, the protein load can be close to the maximum dynamic binding capacity.

In one aspect, the method of the present invention comprises the steps of selecting a conventional chromatography media to be used depending on the nature of antibody provided for separation, and optimizing the separation and removal of glycosylation isomers with the selected chromatography media.

The step of selecting a chromatography media may be accomplished as follows: a crude antibody product to be purified is first loaded on any two or more types of chromatography media under the above salt concentration and pH conditions to thereby select a chromatography media which adsorbs more antibody having a sugar chain attached only to the glycosylation consensus region. Further, these chromatography media may optionally be eluted with an eluent whose salt concentration is reduced in a stepwise or continuous fashion, and eluents passing through the media are each measured for the levels of glycosylation isomers and/or an antibody having a sugar chain attached only to the glycosylation consensus region, whereby a chromatography media giving an eluent with reduced levels of glycosylation isomers and rich in the antibody having a sugar chain attached only to the glycosylation consensus region may be selected as a chromatography media which is more excellent in the separation of glycosylation isomers.

In the step of optimizing the separation and removal of glycosylation isomers with the selected chromatography media, conditions are considered and selected, including the composition, concentration and pH of a buffer used for loading, the type and concentration of a salt to be added, the amount of a crude antibody product loaded on the chromatography media, the composition, concentration and pH of a buffer used for washing, the frequency of washing and the volume of a wash buffer, the composition, concentration and pH of a buffer used for elution, the type and concentration of a salt to be added, and how to change them, and whether glycosylation isomers are removed by adsorption or flow through. In the optimization step of these conditions, individual parameters may be optimized one by one, or alternatively, statistical analysis procedures such as the design of experiments may be used to select the optimal conditions including interactions among several parameters.

Techniques for antibody purification involve various steps. Most processes for antibody purification are conducted in two or more steps using different chromatography modes, but purification processes are usually often constructed using three steps of chromatography. For example, two or more of Protein A affinity chromatography, cation exchange chromatography, anion exchange chromatography, mixed-mode chromatography, hydrophobic interaction chromatography and so on are used in combination. The method of the present invention may be integrated into any step of conventional chromatography in these antibody purification processes. In more detail, in a purification process comprising Protein A affinity chromatography in the first step, cation exchange chromatography in the second step and hydrophobic interaction chromatography in the third step, the method of the present invention may be used as hydrophobic interaction chromatography in the third step. Likewise, in the case of a purification process comprising Protein A affinity chromatography in the first step, mixed-mode chromatography in the second step and hydrophobic interaction chromatography in third step, the method of the present invention may be used as hydrophobic interaction chromatography in the third step. Likewise, in the case of a purification process comprising Protein A affinity chromatography in the first step, hydrophobic interaction chromatography in the second step and mixed-mode chromatography in the third step, the method of the present invention may be used as hydrophobic interaction chromatography in the second step. Further, in the case of a purification process comprising Protein A affinity chromatography in the first step, anion exchange chromatography in the second step and hydrophobic interaction chromatography in third step, the method of the present invention may be used as hydrophobic interaction chromatography in the third step. Moreover, in the sequence of these chromatography steps, the method of the present invention may be used as a mixed-mode chromatography step in addition to hydrophobic interaction chromatography or in place of hydrophobic interaction chromatography.

The method of the present invention enables the efficient removal of glycosylation isomers and the provision of a purified antibody composition containing glycosylation isomers reduced to the desired content. By the method of the present invention, the ratio of glycosylation isomers relative to the total antibody in the purified antibody composition after purification can be 10% or less, more desirably 5% or less, 4% or less, 3% or less, 2% or less, 1% or less, 0.5% or less, or 0.2% or less. Moreover, the ratio of glycosylation isomers relative to the total antibody in the crude antibody product before purification may be 50% or more, 20% or more, 10% or more, or 5% or more. The method of the present invention may be a method for obtaining an antibody with reduced content of glycosylation isomers in a step yield of 20% or more, 40% or more, or 50% or more.

The content and ratio of glycosylation isomers and the purification yield in the final purified antibody composition or the antibody composition after purification may be achieved by adjusting the above chromatography parameters. Preferably, these parameters are optimized such that glycosylation isomers are reduced to any levels and a purification yield acceptable for antibody production is obtained.

The status of glycosylation isomer removal may be confirmed by high-performance liquid chromatography (HPLC) or ultra-high performance liquid chromatography (UHPLC) for analysis and evaluation purposes. In these analyses, a media with an average particle size of 15 µm or less is used, and glycosylation isomers can be separated by chromatography at ultra-high flow rate and at ultra-high pressure. For example, columns suitable for this purpose include a TSKgel Butyl-NPR column (average particle size: 2.5 µm, Tosoh Corporation, Japan), a TSKgel Phenyl-5PR column (average particle size: 10 or 13 µm, Tosoh Corporation, Japan), a TSKgel Ether-5PW column (average particle size: 10 µm, Tosoh Corporation, Japan), a TSKgel BioAssist Phenyl column (average particle size: 10 µm, Tosoh Corporation, Japan), a Protein-Pak Hi Res HIC column (Waters), a BioPro HIC column (average particle size: 2.3 or 4 µm, YMC Co., Ltd., Japan), a Proteomix HIC column (average particle size: 1.7 or 5 µm, M&S Instruments Inc., Japan), an AdvanceBio HIC column (average particle size: 3.5 µm, Agilent), an MAbPac HIC-10 LC column (average particle size: 5 µm, Thermo Fisher), an MabPac HIC-20 LC column (average particle size: 5 µm, Thermo Fisher), an MabPac HIC-Butyl LC column (average particle size: 5 µm, Thermo Fisher), a Shimpack Bio-HIC column (average particle size: 4 µm, Shimadzu Corporation, Japan), Shodex HIC PH-814 (average particle size: 10 µm, Shoko Co., Ltd., Japan), COSMOSII, HIC (average particle size: 5 µm, Nacalai Tesque Inc., Japan), etc.

The purified antibody composition thus purified by the method of the present invention can be used as an active ingredient of antibody drugs for use as therapeutic, prophylactic or diagnostic agents for various diseases in humans and animals.

### Examples

The present invention will be further described in more detail by way of the following examples, which are not intended to limit the scope of the present invention.

### Example 1

Preparation and compositional evaluation of crude antibody product (culture supernatant) containing antibodies having sugar chains linked to sites other than the glycosylation consensus region

The glycosylated antibody used was the humanized anti-human DLK-1 monoclonal antibody shown in WO2014/054820 having H and L chains (H chain: SEQ ID NO: 64, L chain: SEQ ID NO: 72 in the present application) (hereinafter referred to as "anti-hDLK-1 antibody"). This antibody has a glycosylation consensus sequence in the variable region of its light chain. For this reason, when animal cells or others are engineered to express this gene, there is a possibility that these cells will produce a sugar chain composition containing glycosylation isomers as a contaminant.

The CHO cell line DG44 was transformed with an expression vector carrying a gene encoding the amino acid sequence of the anti-hDLK-1 antibody to prepare a stable expression cell line pool, DGC8-R-T11-14.2d. This cell line pool was used and cultured in a DASGIP^{®} Parallel Bioreactor System (Eppendorf) bioreactor on a 1 L scale. The culture was conducted using a serum-free completely chemical synthetic medium by the fed-batch mode for 13 days under conditions of pH 7 and 34°C to 37°C. This culture supernatant was purified by Protein A chromatography (with a MabSelect SuRe media (Cytiva)) to obtain an antibody composition.

### <HIC-HPLC analysis of antibody composition>

The resulting antibody composition was subjected to HIC-HPLC analysis under the following conditions.
HPLC apparatus: a Prominence HPLC System (Shimadzu Corporation, Japan)
Analytical column: a TSKgel Butyl-NPR column (Tosoh Corporation, Japan, 0014947; 4.6 mm × 3.5 cm, average particle size: 2.5 µm)
Mobile phase A: 0.1 M sodium phosphate buffer (pH 7.0) containing 2.3 M ammonium sulfate
Mobile phase B: 0.1 M sodium phosphate buffer (pH 7.0)
Analysis conditions: injected at a protein concentration of 2 mg/mL × 10 µL, gradient: 0 to 3 minutes; 0% B, 3 to 15 minutes; 0% to 100% B, 15 to 20 minutes; 100% B, flow rate: 0.5 mL/min, detection wavelength: 220 nm or 280 nm

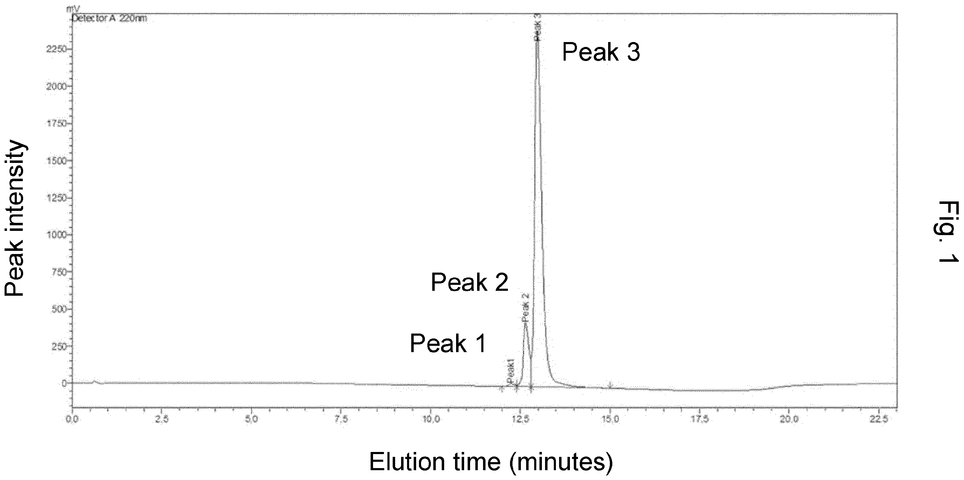

As shown in Figure 1, three peaks were separated, i.e., a main peak (Peak 3) was detected at an elution time of around 13 minutes, and Peak 2 and Peak 1 were detected at elution times of around 12.5 minutes and 12.0 minutes, respectively. Peak 3 was an antibody having a sugar chain linked only to the glycosylation consensus region, while Peak 2 was deemed to be a glycosylation isomer having additional one sugar chain linked to the antibody of Peak 3, and Peak 1 was deemed to be a glycosylation isomer having additional two sugar chains linked to the antibody of Peak 3 (see Example 2). This analysis result indicates that the crude antibody product obtained by culturing cells of the above pool was confirmed to contain about 10% of glycosylation isomers.

### Example 2

Preparation and glycosylation status confirmation of crude antibody product (culture supernatant) containing antibodies having sugar chains linked to sites other than the glycosylation consensus region

An expression vector (pFUSE) carrying gene sequences encoding the amino acid sequences of the heavy and light chains of the anti-hDLK-1 antibody, and an expression vector (pFUSE) encoding a mutant sequence (Asn-Ser-Ala) comprising a point mutation introduced into the third amino acid in the glycosylation consensus sequence (Asn-Ser-Ser) in the light chain of the anti-hDLK-1 antibody and encoding the amino acid sequence of the heavy chain of the anti-hDLK-1 antibody were each used to transform ExpiCHO cells to cause the transient expression of each antibody protein. Their culture supernatants were purified by Protein A affinity chromatography, and the resulting crude antibody products were treated with peptide-N-glycosidase F (PNGase F) or not treated with PNGase F, and then analyzed by reducing SDS polyacrylamide gel electrophoresis (SDS-PAGE) (silver staining). The unmutated anti-hDLK-1 antibody is designated as NSS antibody, while the anti-hDLK-1 antibody whose light chain glycosylation consensus sequence was mutated is designated as NSA antibody.

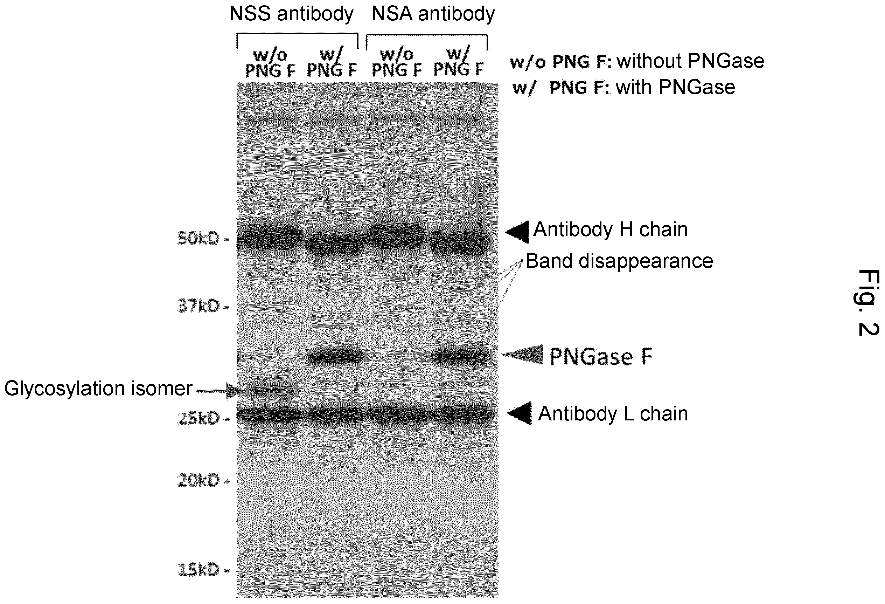

As a result, as shown in Figure 2, in the NSS antibody, a band with a molecular weight of greater than 25 kDa was found above the 25 kDa light chain band under non-PNGase F-treated conditions, but this band disappeared upon PNGase F treatment. This means that the NSS antibody was confirmed to have a sugar chain linked to the light chain having a consensus sequence other than the glycosylation consensus region in the antibody. On the other hand, this band was not found in NSA, regardless of the presence or absence of PNGase F treatment. These results indicate that a transient expression-derived crude product of the humanized anti-DLK-1 monoclonal antibody (NSS antibody) contains glycosylation isomers having sugar chains attached to the light chain.

### Example 3

### Separation of glycosylation isomers with hydrophobic interaction chromatography media (1)

A culture supernatant containing the anti-hDLK-1 antibody prepared in the same manner as shown in Example 1 was purified by Protein A affinity chromatography to obtain a crude product of this antibody containing about 10% of glycosylation isomers. To this crude antibody product, sodium chloride was added to give a final concentration of 1.0 M, and the crude antibody product was then adjusted to pH 5.0 and provided for use as a loading sample on a chromatography media.

Using this crude antibody product, its adsorption properties to a hydrophobic interaction chromatography column (column volume: 5 mL) filled with Capto Butyl (average particle size: 90 µm, Cytiva) were evaluated in bind-elute mode. The amount of the sample loaded on the column was set to 15 mg/mL as the protein load per unit volume of the media, and the flow rate was set to 300 cm/h. The chromatography was conducted under the following mobile phase conditions.
Mobile phase A: 20 mM sodium citrate-15 mM Tris buffer (pH 5.0) containing 1 M sodium chloride
Mobile phase B: 20 mM sodium citrate-15 mM Tris buffer (pH 5.0)
   1. Equilibration: 100% mobile phase A; 10 column volumes (CV)
   2. Loading: sample of the crude antibody product
   3. Washing: 100% mobile phase A; 10 CV
   4. Linear gradient elution: from 0% mobile phase B to 100% mobile phase B in 40 CV
   5. Column washing: 1 M sodium hydroxide; 5 CV

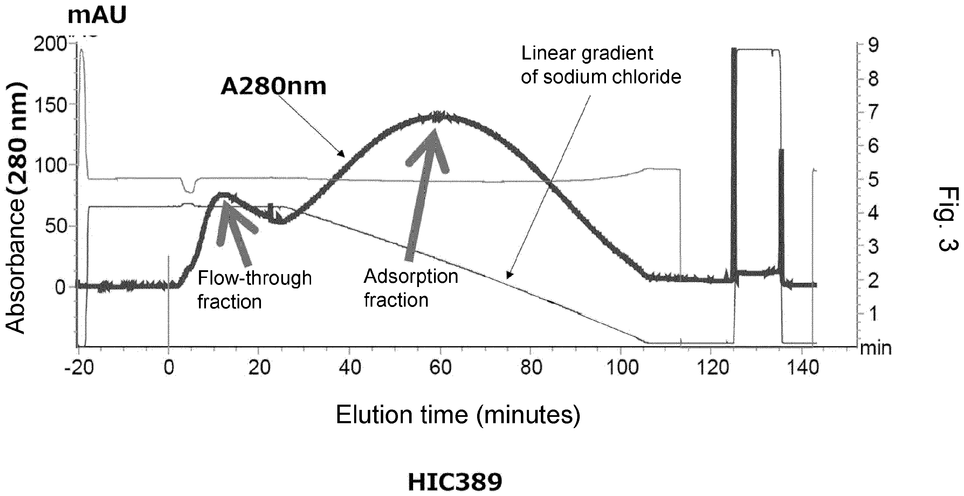

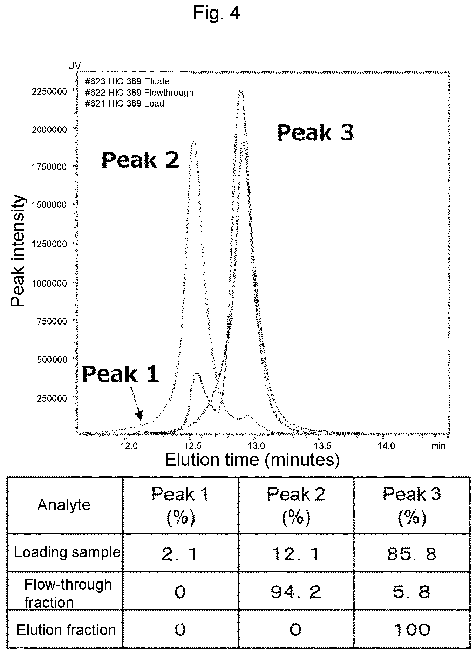

Figure 3 shows an elution chromatogram (HIC 389). This result is indicative of antibody component adsorption to the Capto Butyl media under linear gradient conditions. The loading sample on the column, the flow-through fraction from the Capto Butyl column, and the adsorption fraction to the same column were evaluated by HIC-HPLC analysis as shown in Example 1. The results obtained are shown in Figure 4. Glycosylation isomers contained at 2.1% (Peak 1) and 12.1% (Peak 2) in the loading sample were removed into the flow-through fraction (HIC 389 Flow-through), and a glycosylation isomer-free antibody composition of 100% purity (antibody having no sugar chains attached to sites other than the glycosylation consensus region) (Peak 3) was obtained in the column adsorption fraction (HIC 389 Elute).

These results indicate that glycosylation isomers can be separated by using a hydrophobic interaction chromatography media (Capto Butyl). It is indicated that upon use of the selected chromatography media, glycosylation isomers are efficiently removed into the flow-through fraction, and a high-purity purified antibody composition is obtained in the adsorption fraction.

### Example 4

### Separation of glycosylation isomers with hydrophobic interaction chromatography media (2)

A culture supernatant containing the humanized anti-human DLK-1 monoclonal antibody prepared in the same manner as shown in Example 1 was purified by Protein A affinity chromatography to obtain a crude product of this antibody containing about 10% of glycosylation isomers. To this crude antibody product, sodium chloride was added to give a final concentration of 1.0 M, and the crude antibody product was then adjusted to have a pH of 4.5, 4.7 or 5.0 and a conductivity of 70 mS/cm or less and provided for use as a loading sample on a chromatography media.

Using this crude antibody product, its adsorption properties to a hydrophobic interaction chromatography column (column volume: 5 mL) filled with Capto Butyl (average particle size: 90 µm, Cytiva) or POROS Benzyl Ultra (average particle size: 50 µm, Thermo Fisher) were evaluated in bind-elute mode. The amount of the protein sample loaded on each column was set to 20 mg/mL as the protein load per unit volume of the media, and the flow rate was set to 300 cm/h. The chromatography was conducted under the following mobile phase conditions.
Mobile phase A: 20 mM sodium citrate buffer (pH 4.5, 4.7 or 5.0) containing 1 M sodium chloride
Mobile phase B: 20 mM sodium citrate buffer (pH 4.5, 4.7 or 5.0)
   1. Equilibration: 100% mobile phase A; 5 CV
   2. Loading: sample solution
   3. Washing: 100% mobile phase A; 8 CV
   4. Washing 1: 100% mobile phase A; 5 CV
   5. Washing 2: 100% mobile phase B + 0.8 M sodium chloride; 5 CV
   6. Elution: 100% mobile phase B; 10 CV
   7. Column washing: water (q.s.)
   8. Column washing: 1 M sodium hydroxide (q.s.)

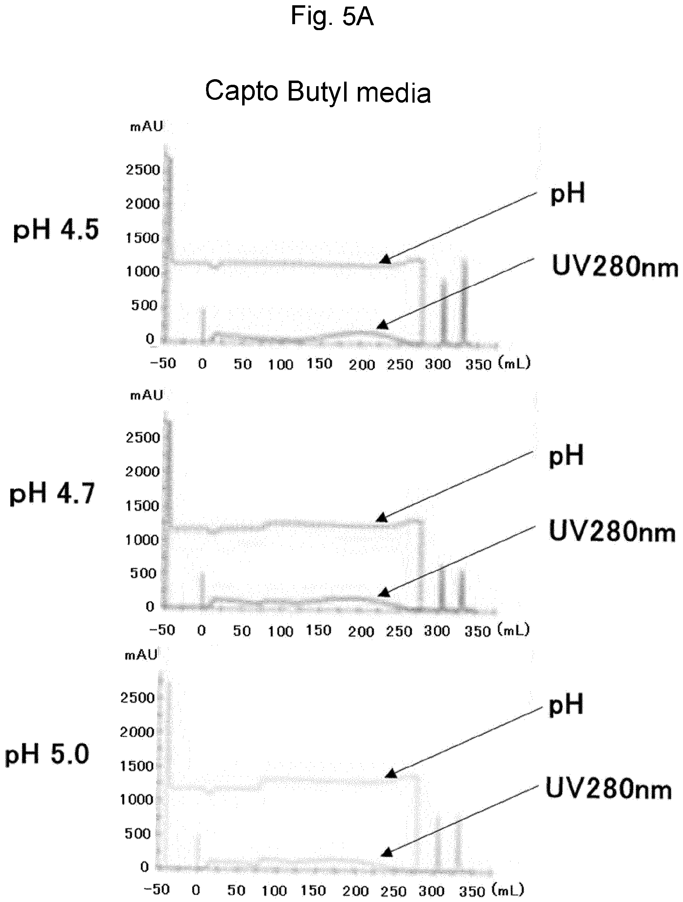

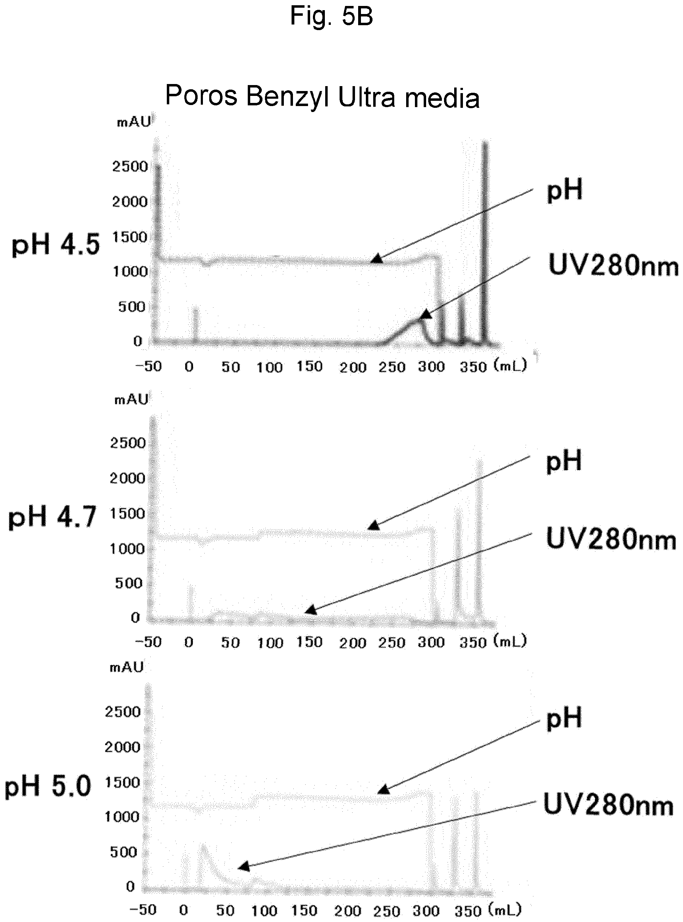

Elution chromatograms from Capto Butyl and POROS Benzyl Ultra are shown in Figure 5A and Figure 5B, respectively. Under all pH conditions, the antibody composition was able to be adsorbed to the Capto Butyl media and then eluted. Good adsorption and elution were also observed for POROS Benzyl Ultra. In more detail, in the graphs shown in Figure 5A and Figure 5B, peaks appearing around 0 to 50 mL in the horizontal axis are antibodies (glycosylation isomers) which were eluted by flowing through the column without being adsorbed, while an antibody eluted as a peak ranging from 50 to 300 mL indicates that an antibody having no sugar chains attached to sites other than the glycosylation consensus region is gradually eluted from the column. In the case of Capto Butyl, antibody elution was observed throughout washing/elution, regardless of pH, thus indicating that the antibody having no sugar chains attached to sites other than the glycosylation consensus region can be obtained with high purity when collecting an intermediate layer of elution. In the case of Poros Benzyl Ultra, the peak was found to vary depending on pH (at pH 4.5, the antibody was well adsorbed and eluted from the column during the second half of elution, whereas at pH 5.0, around 80% of the antibody was not adsorbed, and the antibody was eluted during washing), thus indicating that the component to be eluted can be controlled depending on pH. These results indicate that glycosylation isomers can be separated by using either Capto Butyl or POROS Benzyl Ultra, thus suggesting that separation of glycosylation isomers is possible with a hydrophobic interaction chromatography media. Moreover, this result indicates that hydrophobic interaction chromatography allows the adsorption of an antibody composition and achieves the removal of glycosylation isomers when optimizing the pH, salt concentration and volume of a wash buffer/eluent.

### Example 5

Study on the removal efficiency of glycosylation isomers (purity and yield) in hydrophobic interaction chromatography media, depending on the salt concentration for loading, the salt concentration and pH for washing, and the type of elution buffer.

The Capto Butyl and POROS Benzyl Ultra chromatography media were studied as to whether they were able to remove glycosylation isomers by stepwise elution. For use as a loading solution, the crude antibody product of Example 1 purified by Protein A affinity chromatography was adjusted with 0.5 M Tris to pH 4.5 or pH 5.0. As parameters, the salt concentration of a sample loading solution, the pH and salt concentration of wash buffers (wash buffer I, wash buffer II), and the type and pH of an elution buffer were studied to measure the yield of antibody and the purity of non-glycosylation isomer antibody in the purified fraction (i.e., the purity of antibody having no sugar chains attached to sites other than the glycosylation consensus region) (the content of glycosylation isomers by HIC-HPLC analysis). The test results obtained are shown in Table 3.

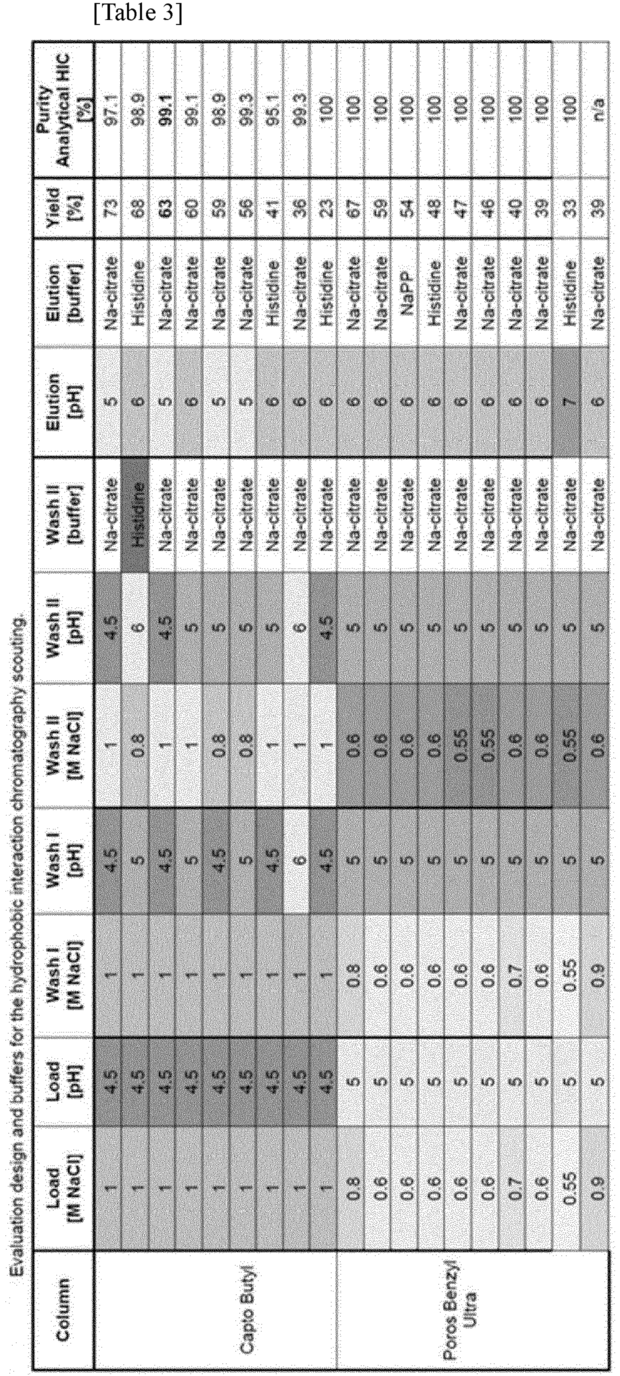

The purification yield with the Capto Butyl media under the respective conditions was 23% to 73%, and the purity of the purified antibody composition (antibody component free from glycosylation isomers) by HIC-HPLC analysis was 97.1% to 99.3%. On the other hand, the purification yield with the POROS Benzyl Ultra media was slightly lower (33% to 67%), but the purity of the purified antibody composition by HIC-HPLC analysis was 100% under all the conditions. In particular, among the conditions used to study the POROS Benzyl Ultra media, conditions where a sample containing 0.8 M sodium chloride was loaded, and the media was washed sequentially with wash buffer I containing 0.8 M sodium chloride and then wash buffer II containing 0.6 M sodium chloride, and eluted with sodium citrate buffer of pH 6 were found to be optimal in terms of purity and yield.

Moreover, the results of this series of tests indicate the following.
(i) The purity is improved with increase in the pH of the wash buffer (wash buffer I and/or wash buffer II).
(ii) The yield is reduced with increase in the pH of the wash buffer (wash buffer I and/or wash buffer II).
(iii) The purity is improved with increase in the pH of the eluent.
(iv) The yield is reduced with increase in the pH of the eluent.
(v) The yield is reduced with decrease in the salt concentration of the loading solution and/or the wash buffer.
(vi) The conditions can be set to achieve 95% or more, ideally 99% or more purity of the antibody composition.

As can be seen from these results, it is indicated that the yield and the amount of glycosylation isomer contamination can be optimized in hydrophobic interaction chromatography media (e.g., Capto Butyl and POROS Benzyl Ultra) when optimizing the salt concentration and pH of a loading solution, the salt concentration and pH of a wash buffer, the volume of the wash buffer, the frequency of washing, the pH for elution and the type of buffer.

### Example 6

Optimization of washing conditions for controlling the rate of glycosylation isomer contamination and the purity and yield of desired product

A culture supernatant containing the anti-hDLK-1 antibody prepared in the same manner as shown in Example 1 was purified by Protein A affinity chromatography to obtain a crude product of this antibody containing about 10% of glycosylation isomers. To this crude antibody product, sodium chloride was added to give a final concentration of 1.0 M, and the crude antibody product was then adjusted as appropriate for its pH and conductivity and provided for use as a loading sample on a chromatography media. Using this crude antibody product and the POROS Benzyl Ultra media, the load mass of the sample and the volumes of wash buffer I and wash buffer II were used as input parameters to evaluate the yield and the purity by HIC-HPLC analysis, which were output parameters.

As a result, it was indicated that the yield was able to be controlled to 40% to 80% with 97% to 100% purity when the protein load per unit volume of the chromatography media was adjusted to 25 g/L or more, and wash buffer I and wash buffer II were adjusted to 5 to 15 column volumes (CV) (Table 4). In particular, the purity of the resulting purified antibody composition tended to be higher when the protein load was 30 g/L or 35 g/L than when the protein load was 25 g/L. Moreover, when increasing the volume of each wash buffer, there was a tendency that the yield of the resulting purified antibody composition was reduced, but its purity was improved. This result indicates that under low protein load conditions, the purity of the purified antibody composition can be increased by increasing the volume of each wash buffer.

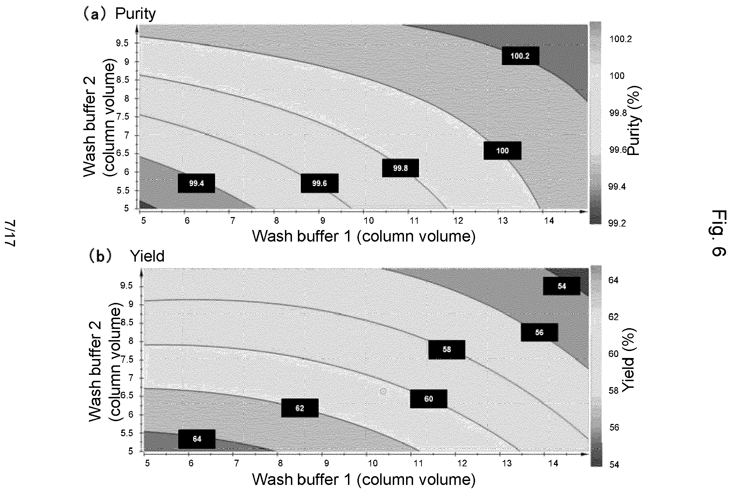

Namely, this result indicates that when the protein load in chromatography is set to a certain value or higher and/or when the volume of each wash buffer is controlled appropriately, the content of glycosylation isomers can be reduced and controlled from about 10% to the range of 3% to 0%. These results were analyzed by the design of experiment (DoE), and the results obtained are shown in Figure 6. The volumes of wash buffer I and wash buffer II and their effect on the yield and purity are expressed in contour chart form, thus indicating that any yield and purity can be controlled by the volumes of wash buffers.

**[Table 4]**

| **Evaluation design and buffers for the hydrophobic interaction chromatography scouting with Poros Benzyl Ultra.** | | | | | | | |
|---|---|---|---|---|---|---|---|
| **Run** | **Run ID** | **Load Mass [g/L]** | **Flow rate [cm/h]** | **Wash I [CV]** | **Wash II [CV]** | **Yield [%]** | **Purity [analytical HIC]** |
| 1 | HIC 464 001 | 25 | 300 | 10 | 7.5 | 73.0 | 98.4 |
| 2 | HIC 464 002 | 30 | 300 | 10 | 5 | 64.0 | 99.6 |
| 3 | HIC 464 003 | 25 | 300 | 15 | 10 | 65.4 | 99.6 |
| 4 | HIC 464 004 | 35 | 300 | 15 | 5 | 50.0 | 100 |
| 5 | HIC 464 006 | 35 | 300 | 10 | 7.5 | 49.4 | 100 |
| 6 | HIC 464 006 | 25 | 300 | 15 | 5 | 69.4 | 99.1 |
| 7 | HIC 464 007 | 30 | 300 | 5 | 7.5 | 62.7 | 99.6 |
| a | HIC 464 008* | 30 | 300 | 10 | 7.5 | 59.5 | 100 |
| 9 | HIC 464 009* | 30 | 300 | 10 | 7.5 | 58.5 | 100 |
| 10 | HIC 464 010 | 30 | 300 | 15 | 7.5 | 54.8 | 100 |
| 11 | HIC 464 011 | 35 | 300 | 5 | 5 | 57.1 | 100 |
| 12 | HIC 464 012 | 35 | 300 | 15 | 10 | 44.4 | 100 |
| 13 | HIC 464 013 | 30 | 300 | 10 | 10 | 56.1 | 100 |
| 14 | HIC 464 014* | 30 | 300 | 10 | 7.5 | 58.2 | 100 |
| 15 | HIC 464 015 | 35 | 300 | 5 | 10 | 48.7 | 100 |
| 16 | HIC 464 016 | 25 | 300 | 5 | 5 | 74.0 | 97.1 |
| 17 | HIC 464 017 | 25 | 300 | 5 | 10 | 66.1 | 99.0 |
| 19 | HIC 467 001 | 40 | 200 | 15 | 5 | 40.4 | n/a |
| 19 | HIC 467 002 | 35 | 200 | 15 | 5 | 46.2 | n/a |

| | | | | | | | |
|---|---|---|---|---|---|---|---|
| * triplicate run | | | | | | | |

### Example 7

Position of glycosylation isomer removal step in purification process and effect of wash buffer volume on purity

A culture supernatant containing the anti-hDLK-1 antibody prepared in the same manner as shown in Example 1 was purified by Protein A affinity chromatography to obtain a crude product of this antibody containing about 10% of glycosylation isomers. This crude antibody product was kept at pH 3 to 4 for a certain period of time and neutralized for use as a loading sample on a chromatography media.

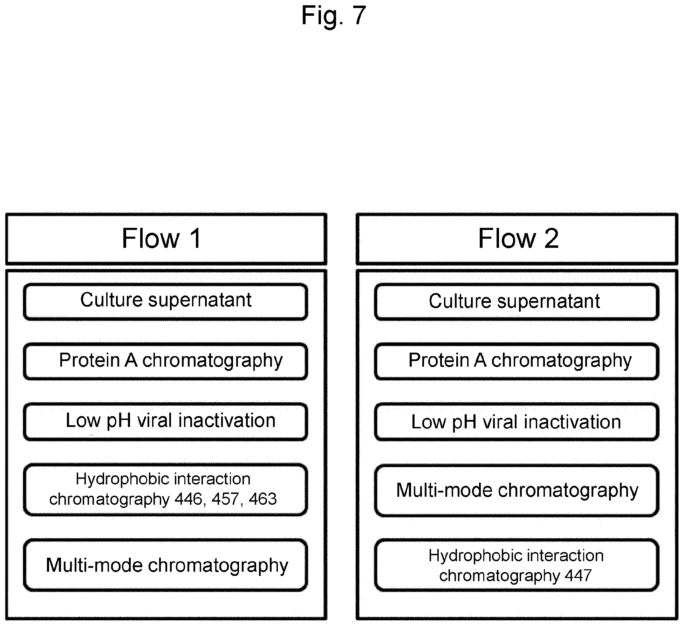

This crude antibody product was purified through the two flows shown in Figure 7, i.e., purified sequentially by hydrophobic interaction chromatography and then mixed-mode chromatography, or sequentially by mixed-mode chromatography and then hydrophobic interaction chromatography. The media used in hydrophobic interaction chromatography was POROS Benzyl Ultra, and the media used in mixed-mode chromatography was Capto MMC. The conditions used for hydrophobic interaction chromatography are as shown below. For ID Nos. HIC 446, HIC 457 and HIC 463, hydrophobic chromatography was followed by mixed-mode chromatography. For ID No. HIC 447, mixed-mode chromatography was followed by hydrophobic chromatography.

### <Conditions for hydrophobic interaction chromatography>

Media: POROS Benzyl Ultra
Column size: 260 mL, bed height: 13.3 cm
Flow rate: 300 cm/h
Protein load per unit volume of the media: 30 g/L
Chromatography conditions:
   1. Equilibration: 20 mM sodium citrate (pH 5.0) containing 0.8 M sodium chloride; 5 CV
   2. Loading: sample of the crude antibody product
   3. Washing 1: 20 mM sodium citrate (pH 5.0) containing 0.8 M sodium chloride; 6 CV (HIC 446/HIC 447), 8 CV (HIC 457) or 15 CV (HIC 463)
   4. Washing 2: 20 mM sodium citrate (pH 5.0) containing 0.6 M sodium chloride; 5 CV
   5. Elution: 20 mM sodium citrate (pH 6.0); 6.3 CV
   6. Column washing: 1 M aqueous sodium hydroxide; 3 CV

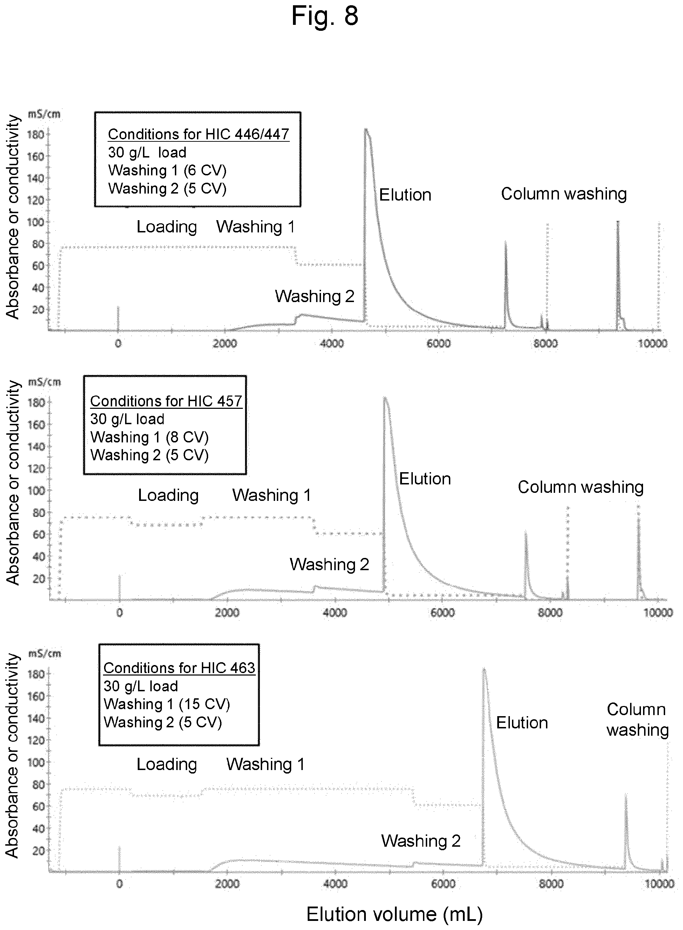

The yield and purity of the purified antibody compositions obtained in the step of hydrophobic interaction chromatography are shown in Table 5 and Figure 8. In either purification flow, the yield was 60% or more, and the purity by HIC-HPLC analysis (which represents the amount of glycosylation isomer contamination) was 99% or more. Moreover, it was indicated that the yield and purity were able to be controlled depending on the volume used in Washing 1 (6 to 15 CV). Namely, the composition with about 90% antibody purity was found to improve its purity to around 98% when washed with 5 CV, around 99% when washed with 8 CV, and around 99.5% when washed with 15 CV

**[Table 5]**

| | Results of the scale-up run of the HIC chromatography. | | | | | |
|---|---|---|---|---|---|---|
| **Run** | **Load amount** | | **Yield** | | **Monomer content** | **Purity analytical HIC** |
| **ID no.** | **[g/L Resin] acc. UV280** | **[g] acc. UV280** | **[g] acc. UV280 ace. UV280** | **[%] acc. UV280** | **[%] ace. SEC-HPLC** | **[%] acc. HIC** |
| HIC 446 | 30 | 7800 | 6113 | 78 | 99.7 | 97.6 |
| HIC 447 | 30 | 7917 | 5671 | 72 | 99.8 | 98.3 |
| HIC 457 | 30 | 7834 | 5650 | 72 | 99.7 | 98.7 |
| HIC 463 | 30 | 7834 | 4923 | 63 | 99.8 | 99.6 |

### Example 8

### Purification process including glycosylation isomer removal step (POROS Benzyl Ultra)

A culture supernatant containing the anti-hDLK-1 antibody prepared in the same manner as shown in Example 1 was obtained in a volume of about 200 L. The crude antibody product was confirmed to contain about 10% of glycosylation isomers.

The culture supernatant was filtered and then subjected to Protein A affinity chromatography (MabSelect SuRe 10 L; Cytiva), low pH viral inactivation, hydrophobic interaction chromatography with a POROS Benzyl Ultra media (Thermo Fisher, 10 L), mixed-mode chromatography with a Capto adhere media (Cytiva, 10 L), viral filtration, replacement with formulation buffer by tangential flow filtration (TFF), sterile filtration and other steps to obtain a high-purity purified antibody composition.

For POROS Benzyl Ultra chromatography, the protein load per unit volume of the media was adjusted to 35 g/L, 30 g/L and 16 g/L, and three chromatography runs were conducted. The conditions used for the POROS Benzyl Ultra chromatography step, and the results of the POROS Benzyl Ultra chromatography step are shown in Table 6 and Table 7, respectively.

**[Table 6]**

| Parameter | Conditions |
|---|---|
| Column | Media volume: 10 L, bed height: 20 cm |
| Equilibration | 20 mM sodium citrate buffer (pH 5.0) containing 0.8 M sodium chloride, 4 CV |
| Loading solution | Protein concentration: 6.71 mg/mL (total volume: about 125 kg, total protein: 841 g) |
| Load per unit volume of media | Load: 35 g/L-resin (loading volume: about 52 L), 30 g/L-resin (loading volume: about 45 L), 16 g/L-resin (loading volume: about 24 L) |
| Washing 1 | 20 mM sodium citrate buffer (pH 5.0) containing 0.8 M sodium chloride, 15 CV |
| Washing 2 | 20 mM sodium citrate buffer (pH 5.0) containing 0.6 M sodium chloride, 5CV |
| Elution | 20 mM sodium citrate buffer (pH 6.0), 10 CV |
| Washing 3 | 1 M aqueous sodium hydroxide |

**[Table 7]**

| | Run 1 | Run 2 | Run 3 |
|---|---|---|---|
| Load | 35 g/L | 30 g/L | 16 g/L |
| Yield | 54% | 58% | 77% |
| Purity | 100% | 100% | 89.74% |

The loads in Run 1 and Run 2 were each in an appropriate range above the predetermined amount, whereas the load in Run 3 was as low as 16 g/L. As a result, the yield in Run 3 was 77%, but a low value of 89.74% was obtained for HIC-HPLC purity, which represents the amount of glycosylation isomer contamination. Thus, only the fractions from Run 1 and Run 2 were combined and provided for the subsequent chromatography step.

The purified antibody composition finally obtained through all the steps was obtained with a total yield of 46%. Moreover, the purity of the purified antibody composition by HIC-HPLC analysis was 100%, thus indicating that the purified antibody composition contained no glycosylation isomers. In this way, a purified antibody composition with sufficiently reduced levels of glycosylation isomers was able to be obtained even under chromatography conditions for antibody. Moreover, the protein load on the chromatography media was also shown to be important in the removal rate of glycosylation isomers.

### Example 9

### Purification process including glycosylation isomer removal step (Capto Butyl)

A culture supernatant containing the anti-hDLK-1 antibody prepared in the same manner as shown in Example 1 was obtained. The crude antibody product was confirmed to contain about 10% of glycosylation isomers.

This culture solution was used and purified through the following steps in this order: Protein A affinity chromatography (media: MabSelect SuRe), low pH viral inactivation, hydrophobic interaction chromatography (Capto Butyl media) and mixed-mode chromatography (Capto adhere).

As can be seen from the results shown in Table 8, the rate free from glycosylation isomers was able to be improved to a purity of 99.8% by the hydrophobic chromatography step, thus obtaining the purified antibody composition with a total yield of 25%.

As indicated in this result, it is shown that a highly purified antibody composition can also be obtained even when using any hydrophobic interaction chromatography media other than the POROS Benzyl Ultra media.

**[Table 8]**

| Purification step | Yield | Purity | Conditions, etc. |
|---|---|---|---|
| Culture supernatant | 100% | 90% | - |
| Protein A affinity chromatography | >90% | 89.9% | - |
| Low pH | 100% | 89.9% | - |
| Hydrophobic interaction chromatography (Capto Butyl media) | 37% | 99.8% | Load: 20 mg/mL, washed with 30 CV of 20 mM sodium citrate buffer (pH 4.5) containing 0.8 M sodium chloride, and then eluted with 10 CV of 20 mM sodium citrate buffer (pH 4.5) containing 50 mM sodium chloride and 75 mM arginine hydrochloride |
| Mixed-mode chromatography (Capto adhere media) | >90% | 99.8% | - |
| Total purification process | 25% | 99.8% | - |

### Example 10

Fractionation of glycosylation isomers and measurement of their biological activity

A crude anti-hDLK-1 antibody product containing about 10% of glycosylation isomers obtained in the same manner as shown in Example 1 was precisely fractionated into individual peaks using the HIC-HPLC analysis system shown in Example 1. The fractionation was accomplished in about ten times, and fractions were combined for each peak, followed by buffer replacement with isotonic phosphate buffer. It should be noted that the HIC-HPLC mobile phase conditions used for fractionation were changed as shown below.
Mobile phase A: 50 mM phosphate buffer (pH 7) containing 2.3 M ammonium sulfate
Mobile phase B: 50 mM phosphate buffer (pH 7)

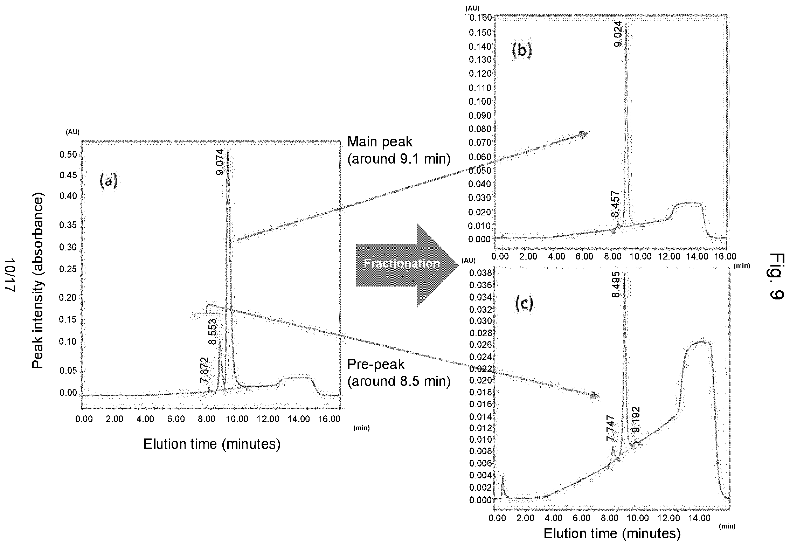

The crude antibody product before fractionation (a) and the purified antibody composition after fractionation (b, c) were analyzed by the HIC-HPLC system shown in Example 1, and the results obtained are shown in Figure 9.

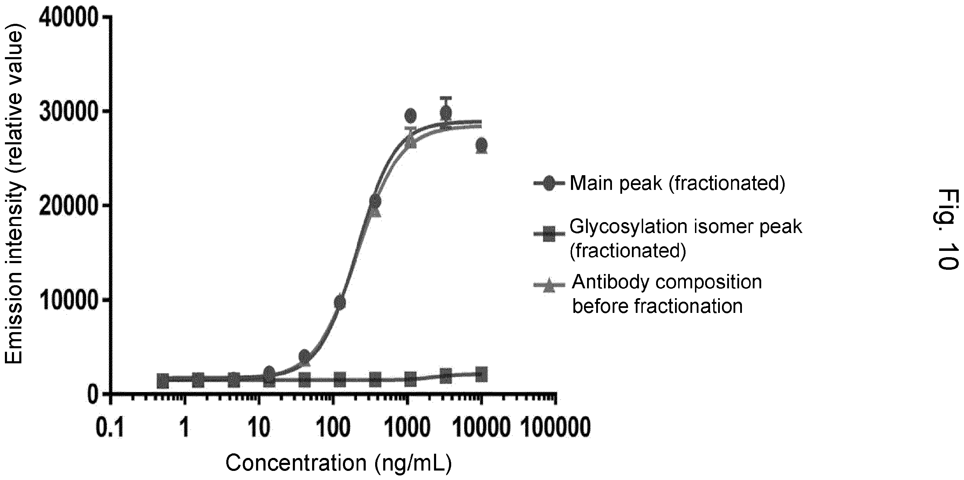

These samples were diluted at a 3-fold common ratio starting from 10 µg/mL, and measured for ADCC activity with an ADCC Reporter Bioassay kit (Promega, G7010) under conditions where the E:T ratio was 6:1 (target cell: HEK293 cell line highly expressing DLK1) and the reaction time was 6 hours. The results obtained are shown in Figure 10. The crude antibody product before fractionation and the main peak after fractionation ((b) in Figure 9) showed substantially the same sigmoid curve, and were confirmed to have equal biological activity. In contrast, the glycosylation isomers isolated by fractionation ((c) in Figure 9) showed almost no signal at all the concentrations tested, and were found to have no biological activity. This result confirms that glycosylation isomers are impurities and are components which should be removed as much as possible as ingredients of biopharmaceuticals.

### Industrial Applicability

The present invention enables the preparation and use of a purified antibody composition with reduced levels of glycosylation isomers having sugar chains attached to sites other than the Fc region glycosylation consensus region.

## Claims

1. A purification method for an anti-hDLK-1 antibody composition, whose heavy chain has an amino acid sequence selected from SEQ ID Nos: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, said method comprising:
Loading a crude antibody product on conventional chromatography to allow an antibody having no sugar chains attached to sites other than the Fc region glycosylation consensus region to be adsorbed to a media of the chromatography; and
treating the media with an eluent to thereby elute the antibody adsorbed to the media to obtain a purified antibody composition,
wherein the content of glycosylation isomers having sugar chains attached to sites other than the Fc region glycosylation consensus region in the resulting purified antibody composition is reduced when compared to the crude antibody product.

2. The purification method according to claim 1, wherein the media of the conventional chromatography is a hydrophobic interaction chromatography media or a mixed-mode chromatography media.

3. The purification method according to claim 1 or 2, wherein the media of the conventional chromatography has an average particle size of 15 µm or more.

4. The purification method according to claim 1 or 2, wherein the media of the conventional chromatography has an average particle size of 20 to 100 µm.

5. The purification method according to any one of claims 1 to 4, wherein the media of the conventional chromatography has a benzyl group or a butyl group.

6. The purification method according to any one of claims 1 to 5, wherein the protein load per unit volume of the media of the conventional chromatography is 20 mg/mL or more.

7. The purification method according to any one of claims 1 to 6, wherein the media of the conventional chromatography is a hydrophobic interaction chromatography media, and wherein said method comprises, after loading the crude antibody product on the media, washing the media with a wash buffer before elution.

8. The purification method according to claim 7, **characterized in that** the salt concentration of the wash buffer is 10 mM or more higher than the salt concentration of the eluent.

9. The purification method according to claim 7 or 8, **characterized in that** the salt concentration of the wash buffer is 0.5 M or more.

10. The purification method according to claim 7 or 8, **characterized in that** the salt concentration of the wash buffer is 1.0 M or more.

11. The purification method according to any one of claims 7 to 10, wherein the pH of the wash buffer is 0.2 Units or more lower than the pH of the eluent and is within the range of pH 4 to 8.

12. The purification method according to any one of claims 7 to 11, wherein the washing is accomplished by passing two or more column volumes of the wash buffer.

13. The purification method according to any one of claims 7 to 12, **characterized in that** the washing and elution are accomplished by using a mobile phase whose pH or/and salt concentration change in a stepwise or linear fashion.

14. The purification method according to any one of claims 7 to 13, wherein the eluent has a salt concentration of 0.5 M or less or contains no salt and has a pH of 5 to 7.

15. The purification method according to any one of claims 1 to 14, wherein said method gives a yield of 20% or more.

16. The purification method according to any one of claims 1 to 15, wherein the ratio of glycosylation isomers relative to the total antibody in the purified antibody composition is 5% or less.

17. The purification method according to claim 16, wherein the ratio of glycosylation isomers relative to the total antibody in the crude antibody product is higher than 5%.

18. The purification method for an anti-hDLK-1 antibody composition according to claim 1, said method comprising:
loading the crude antibody product on conventional chromatography with a media having a benzyl group or a butyl group and having a particle size of 20 to 100 µm;
allowing the antibody having no sugar chains attached to sites other than the Fc region glycosylation consensus region to be adsorbed to the media;
washing the media one or more times with a wash buffer of pH 4 to 6 to remove glycosylation isomers; and
eluting the antibody adsorbed to the media with an eluent of pH 5 to 7 having a salt concentration of 0.5 M or less or containing no salt to obtain a purified antibody composition,
wherein the ratio of glycosylation isomers relative to the total antibody in the purified antibody composition is reduced when compared to the crude antibody product.

19. The purification method for an antibody composition according to claim 1, said method comprising:
loading the crude antibody product adjusted to a salt concentration of 0.5 M or more on conventional chromatography with a media having a benzyl group or a butyl group and having a particle size of 20 to 100 µm to remove glycosylation isomers into a flow-through fraction;
washing the media with a wash buffer; and
eluting the antibody adsorbed to the media with an eluent of pH 5 to 7 having a salt concentration of 0.5 M or less or containing no salt to obtain a purified antibody composition,
wherein the ratio of glycosylation isomers relative to the total antibody in the purified antibody composition is reduced when compared to the crude antibody product.

20. The purification method for an antibody composition according to claim 1, said method comprising:
loading the crude antibody product adjusted to pH 4 to 6 on conventional chromatography with a media having a benzyl group or a butyl group and having a particle size of 20 to 100 µm to remove glycosylation isomers into a flow-through fraction;
washing the media with a wash buffer; and
eluting the antibody adsorbed to the media with an eluent of pH 5 to 7 having a salt concentration of 0.5 M or less or containing no salt to obtain a purified antibody composition,
wherein the ratio of glycosylation isomers relative to the total antibody in the purified antibody composition is reduced when compared to the crude antibody product.

21. The method according to any one of claims 1 to 20, wherein the protein load per unit volume of the media of the conventional chromatography is 20 g/L or more, and the washing is accomplished by passing five or more column volumes of the wash buffer.

22. A production method for an antibody composition, which comprises the purification method according to any one of claims 1 to 21, wherein the ratio of an antibody having no sugar chains attached to sites other than the Fc region glycosylation consensus region relative to the total antibody is 95% or more.

23. A production method for a purified anti-hDLK-1 antibody composition, whose heavy chain has an amino acid sequence selected from SEQ ID Nos: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, said method comprising:
loading a crude antibody product on conventional chromatography with a media having a benzyl group or a butyl group and having a particle size of 20 to 100 µm;
allowing an antibody having no sugar chains attached to sites other than the Fc region glycosylation consensus region to be adsorbed to the media;
washing the media one or more times with a wash buffer containing 0.5 M or more salt to remove glycosylation isomers; and
eluting the antibody adsorbed to the media with an eluent of pH 5 to 7 having a salt concentration of 0.5 M or less or containing no salt to obtain a purified antibody composition,
wherein the ratio of glycosylation isomers relative to the total antibody in the purified antibody composition is reduced when compared to the crude antibody product.

24. An antibody composition produced by the production method according to claim 22 or 23.

25. A method for removing glycosylation isomers having sugar chains attached to sites other than the Fc region glycosylation consensus region from a crude anti-hDLK-1 antibody product, whose heavy chain has an amino acid sequence selected from SEQ ID Nos: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, said method comprising:
Loading the crude antibody product on a hydrophobic interaction chromatography media to allow an antibody having no sugar chains attached to sites other than the Fc region glycosylation consensus region to be adsorbed to the media;
washing the media with a wash buffer; and
treating the media with an eluent to thereby elute the antibody adsorbed to the media to obtain a purified antibody composition.

26. An anti-hDLK-1 antibody composition, whose heavy chain has an amino acid sequence selected from SEQ ID NOs: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, wherein the antibody composition contains an antibody having no sugar chains attached to sites other than the Fc region glycosylation consensus region at a ratio of 95% or more relative to the total antibody.

27. An anti-hDLK-1 antibody, whose heavy chain has an amino acid sequence selected from SEQ ID NOs: 2, 4, 6, 8, 14, 16, 18, 20, 22, 24, 26, 28, 30, 32, 34 and 36, and whose light chain has the amino acid sequence shown in SEQ ID NO: 10 or SEQ ID NO: 12, wherein the antibody has no sugar chains attached to sites other than the Fc region glycosylation consensus region.
